# EUROPEAN PATENT SPECIFICATION

(11) **EP 2 621 499 B1**
(45) Date of publication and mention of the grant of the patent: **22.11.2017**
(21) Application number: 11830072.2
(22) Date of filing: 03.10.2011
(51) Int. Cl.: A61K 31/55, A61K 9/20, A61K 9/48, A61K 9/16, A61P 37/00

(54) **METHODS FOR THE TREATMENT OF ALLERGIC DISEASES**
VERFAHREN ZUR BEHANDLUNG VON ALLERGIEERKRANKUNGEN
MÉTHODES DE TRAITEMENT DE MALADIES ALLERGIQUES

(30) Priority: 01.10.2010 US 388957 P; 01.04.2011 US 201161470837 P
(43) Date of publication of application: 07.08.2013
(73) Proprietor: VentiRx Pharmaceuticals, Inc., Seattle, WA 98101 (US)
(72) Inventor: HERSHBERG, Robert, Seattle WA 98101-1397 (US)
(74) Representative: Cooley (UK) LLP
(86) International application number: PCT/US2011/054652
(87) International publication number: WO 2012/045089

(56) References cited:
- US-A1- 2008 234 251
- US-A1- 2010 029 585
- MØLLER-LARSEN S ET AL: "Association analysis identifies TLR7 and TLR8 as novel risk genes in asthma and related disorders.", THORAX DEC 2008, vol. 63, no. 12, December 2008 (2008-12), pages 1064-1069, XP002720717, ISSN: 1468-3296

## Description

### FIELD OF THE INVENTION

The present invention is directed to a toll-like receptor 8 (TLR8) agonist and its use in allergy treatment or prevention.

### BACKGROUND OF THE INVENTION

Allergy is manifested in a broad array of conditions and associated symptoms, which may be mild, chronic, acute and/or life threatening. These various pathologies include, for example, allergic asthma, allergic rhinitis, atopic dermatitis, severe food allergies, chronic urticaria and angioedema, as well as the serious physiological condition of anaphylactic shock. A wide variety of antigens are known to act as allergens, and exposure to these allergens results in the allergic pathology. Common allergens include, but are not limited to, bee stings, penicillin, various food allergies, pollens, animal detritus (especially house dust mite, cat, dog and cockroach), and fungal allergens.

Allergies are linked to a variety of common and serious chronic respiratory illnesses and symptoms of allergies, in particular nasal congestion and the concomitant nasal airway obstruction, have a negative impact on day to day activities and overall quality of life. Allergen avoidance, pharmacotherapy (e.g., oral and intranasal antihistamines, intranasal glucocorticosteroids, intranasal and oral decongestants, and antileukotrienes), and immunotherapy (subcutaneous allergen-specific immunotherapy, sublingual or intranasal immunotherapies) comprise the standard of care for moderately affected individuals as recommended by the ARIA (Allergic Rhinitis and its Impact on Asthma, 2008) guidelines. However, for some patients, the side-effects of treatment for allergic rhinitis can outweigh the benefits. Treatment with pharmacotherapeutics may be associated with undesirable effects such as central nervous system symptoms of sedation, impairment of cognition, dizziness, and tremor (antihistamines); cardiovascular symptoms of tachycardia, palpitations, and hypertension (decongestants); and other effects such as nasal irritation and bleeding and possible inhibition of bone growth in children (corticosteroids).

In recent years, the therapeutic options for the treatment of allergic rhinitis have increased but for the most part are only moderately efficacious and frequently not satisfying. At the same time, the incidence of allergic rhinitis has been rising worldwide and most rapidly in developed countries. This observation has been attributed to a number of factors including environmental conditions, genetics, and improved hygiene in industrialized countries. Epidemiological data show striking differences in the incidence of allergic diseases in the industrialized compared to the non-industrialized world, suggesting a microbial link to allergic predisposition. The "hygiene hypothesis" proposes that a lack of microbial exposure associated with improved public health can heighten allergic immune tone on mucosal or skin surfaces and may reflect an alteration in the local innate immune response triggered by microbial ligands. Thus, there exists a strong need to develop treatments for allergic diseases.

Stimulation of the immune system, which includes stimulation of either or both innate immunity and adaptive immunity, is a complex phenomenon that can result in either protective or adverse physiologic outcomes for the host. A common immune response theme has emerged wherein so-called pattern recognition receptors (PRR) are employed that recognize invariant structures within microbial agents. In recent years there has been increased interest in the mechanisms underlying innate immunity, which is believed to initiate and support adaptive immunity. This interest has been fueled in part by the recent discovery of a family of highly conserved pattern recognition receptor proteins known as Toll-like receptors (TLRs) believed to be involved in innate immunity as receptors for pathogen associated molecular patterns (PAMPs). Compositions and methods useful for modulating innate immunity are therefore of great interest, as they may affect therapeutic approaches to conditions involving autoimmunity, inflammation, allergy, asthma, graft rejection, graft versus host disease (GvHD), infection, cancer, and immunodeficiency.

Stimulation of an innate immune response via TLRs may provide therapeutic benefit in allergic disease via down regulation of the adaptive immune response. Toll-like receptors (TLRs) are a family of type I transmembrane proteins whose *in vivo* activation initiates an innate immune response involving specific cytokines, chemokines and growth factors. While all TLRs can activate certain intracellular signaling molecules such as nuclear factor kappa beta (NF-kB) and mitogen activated protein kinases (MAP kinases), the specific set of cytokines and chemokines released appears to be unique for each TLR. TLR4 which recognizes lipopolysaccharides (LPS) on the cell wall of gram negative bacteria was one of the first of the family of TLRs to be evaluated as a therapeutic target in allergic disease. MPL®, (a biological fermentation product from Salmonella Minnesota that contains various species of LPS) has been successfully combined with injected ragweed allergen to reduce the number of antigen injections required for immunotherapy.

TLR7, 8, and 9 comprise a subfamily of TLRs which are located in endosomal or lysosomal compartments of immune cells such as dendritic cells and monocytes. In contrast to TLR7 and 9 which are highly expressed on plasmacytoid dendritic cells (pDC), TLR8 is mainly expressed on myeloid DC (mDC) and monocytes. CpG oligonucleotides, which stimulate TLR9, have been conjugated to ragweed antigen (Tolamba®) in a similar strategy to provide enhanced desensitization following injection. Results from the initial trials were encouraging; however, results from subsequent studies of the product were equivocal. On the other hand, TLR9 agonists have been added to an arsenal of anti-cancer drugs as they increase antigen presentation and booster T and B cell responses. In addition, intranasal administration of MPL in the absence of antigen showed minimal improvement in nasal symptom scores in a small Phase 1 study. Recently, TLR8, which recognizes single-stranded viral RNA as its natural ligand, has emerged as an interesting target in the treatment of allergic disease. Genetic association studies report linkages between TLR8 and asthma and allergic rhinitis. While the precise molecular mechanisms underlying the potential clinical benefit of a TLR agonist in allergy are not completely understood, laboratory studies have underscored the importance of IL-12 and IL-10 in the anti-allergic efficacy. In this regard, activation of TLR8 stimulates production of both IL-12 and IL-10 in human monocytes and myeloid dendritic cells. Unlike other TLRs, TLR8 (and its closest relative TLR7) can recognize synthetic small molecules (MW<500) making it an attractive target for pharmaceutical development. Agonists of TLR8 stimulate the production of various inflammatory cytokines including interleukin-6, interleukin-12, tumor necrosis factor-alpha, and interferon-gamma. Such agonists also promote the increased expression of co-stimulatory molecules such as CD40, CD80, and CD86, major histocompatibility complex molecules, and chemokine receptors. The type I interferons, IFNα and IFNβ, are also produced by cells upon activation with TLR8 agonists.

Small, low-molecular weight (less than 400 Daltons) synthetic imidazoquinoline compounds which resemble the purine nucleotides adenosine and guanosine were the first TLR7 and TLR8 agonists to be identified. A number of these compounds have demonstrated anti-viral and anti-cancer properties. For example, the TLR7 agonist imiquimod (ALDARA™) was approved by the U.S. Food and Drug Administration as a topical agent for the treatment of skin lesions caused by certain strains of the human papillomavirus. Imiquimod may also be useful for the treatment of primary skin cancers and cutaneous tumors such as basal cell carcinomas, keratoacanthomas, actinic keratoses, and Bowen's disease. The TLR7/8 agonist resiquimod (R-848) is being evaluated as a topical agent for the treatment of human genital herpes.

Howbert et al. US 2010/029585 A1 describes benzo[b]ezapine TLR7 and TLR8 agonists for use in treating certain skin conditions, such as atopic dermatitis. Doherty et al. US 2008/234251 A1 describes TLR7 and/or TLR8 ligands for use in treating various conditions, including asthma and allergies.

### SUMMARY OF THE INVENTION

The invention is defined by the appended claims.

The present disclosure is directed generally to a benzo[b]azepine TLR8 agonist for use in the treatment of allergic diseases, such as allergic rhinitis. Specifically, the present invention is directed to the benzo[b]azepine TLR8 agonist VTX-378 or a salt thereof. VTX-1463 is an intranasal formulation of VTX-378.

The invention provides a pharmaceutical composition for use in a method for treating, ameliorating, or preventing an allergic disease in a subject in need thereof, the pharmaceutical composition comprising VTX-378 represented by the structure: 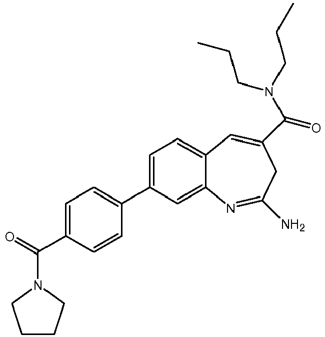 or a pharmaceutically acceptable salt or hydrate thereof, wherein VTX-378 or a pharmaceutically acceptable salt or hydrate thereof is administered intranasally.

Preferably, VTX-378 or a salt thereof is formulated at a concentration of from about 0.5 mg/ml to about 50 mg/ml, from about 1 mg/ml to about 40 mg/ml, or from about 2 mg/ml to about 15 mg/ml. In certain embodiments, VTX-378 or a salt thereof is formulated at a concentration of from about 0.5 mg/ml to about 10 mg/ml, from about 0.5 mg/ml to about 8 mg/ml, from about 0.5 mg/ml to about 6 mg/ml, from about 0.5 mg/ml to about 4 mg/ml, or from about 0.5 mg/ml to about 2 mg/ml. In certain embodiments, VTX-378 or a salt thereof is formulated at a concentration of about 0.5 mg/ml, about 1 mg/ml, about 2 mg/ml, about 4 mg/ml, about 6 mg/ml, about 8 mg/ml, about 10 mg/ml, about 15 mg/ml, about 20 mg/ml, about 25 mg/ml, about 30 mg/ml, about 40 mg/ml, or about 50 mg/ml. Preferably, the formulation comprises about 1-30%, 5-15%, or 5-10% weight/volume (w/v) of a cyclodextrin, preferably a β-cyclodextrin, and most preferably sulfobutylether β-cyclodextrin. In certain embodiments, the formulation comprises 1%, 5%, 10%, 15%, 20%, 25%, or 30% w/v of a cyclodextrin, preferably a β-cyclodextrin, and most preferably sulfobutylether β-cyclodextrin. In a particular embodiment, the formulation is an aqueous solution comprising VTX-378 or a salt thereof at a concentration of at least 2 mg/ml. In a further embodiment, the formulation comprises 15% w/v of a cyclodextrin, preferably a β-cyclodextrin, and most preferably sulfobutyl ether β-cyclodextrin. In preferred embodiments, the formulation is suitable for injection in a mammal, preferably a human.

The present invention provides a pharmaceutical composition comprising VTX-378 or a salt thereof for use in treating allergic diseases, such as allergic rhinitis, said composition being in a form suitable for nasal administration.

It has been unexpectedly discovered that VTX-378 can control both the rapid onset of symptoms and longer term immunomodulation. As such, the present invention relates to a pharmaceutical composition for use in treating, ameliorating, or preventing seasonal or perennial allergic diseases.

The dosing regimen that can be used in the methods of the invention includes, but is not limited to, daily, three times weekly (intermittent), two times weekly, weekly, or every 14 days. In certain embodiments, dosing regimen includes, but is not limited to, monthly dosing or dosing every 6-8 weeks. VTX-378 of the present invention is administered by intranasal administration weekly or biweekly alone or in combination with a suitable treatment modality for the treatment of allergic diseases in a subject, preferably a human subject.

The present disclosure also provides a pharmaceutical pack or kit comprising one or more containers filled with VTX-378 or a salt thereof in a form suitable for nasal administration or other routes of administration.

Unless otherwise defined, all technical and scientific terms used herein have the same meaning as commonly understood by one of ordinary skill in the art to which this invention belongs. In the specification, the singular forms also include the plural unless the context clearly dictates otherwise. In the case of conflict, the present specification, including definitions, will control.

Other features and advantages of the invention will be apparent from the following detailed description and claims.

### BRIEF DESCRIPTION OF DRAWINGS

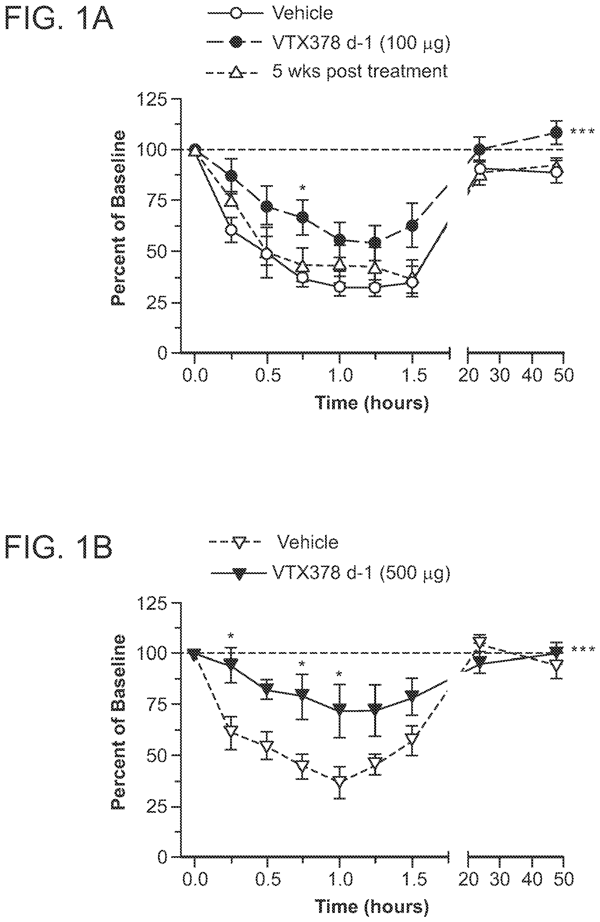

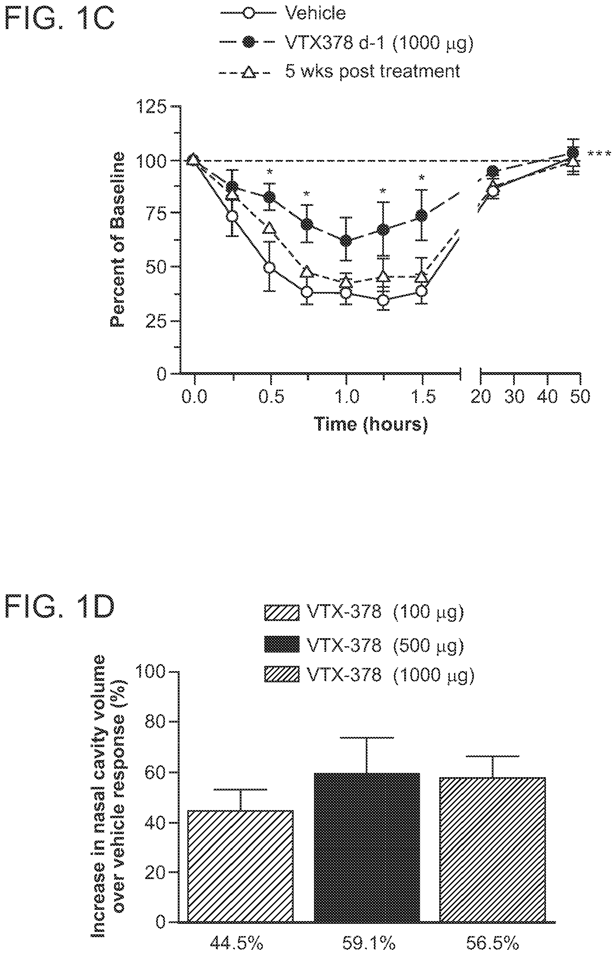

**Figure 1** is four graphs establishing that one day pretreatment with VTX-378 reduces nasal congestion. Pretreatment with a single dose of VTX-378 at 100 ug (A), 500 ug (B) and 1000 ug (C) administered intranasally one day prior to ragweed challenge significantly attenuated allergic responses in a ragweek-sensitized beagle dog model of allergic rhinitis. (D) shows the increase in nasal cavity volume over vehicle response (5%).

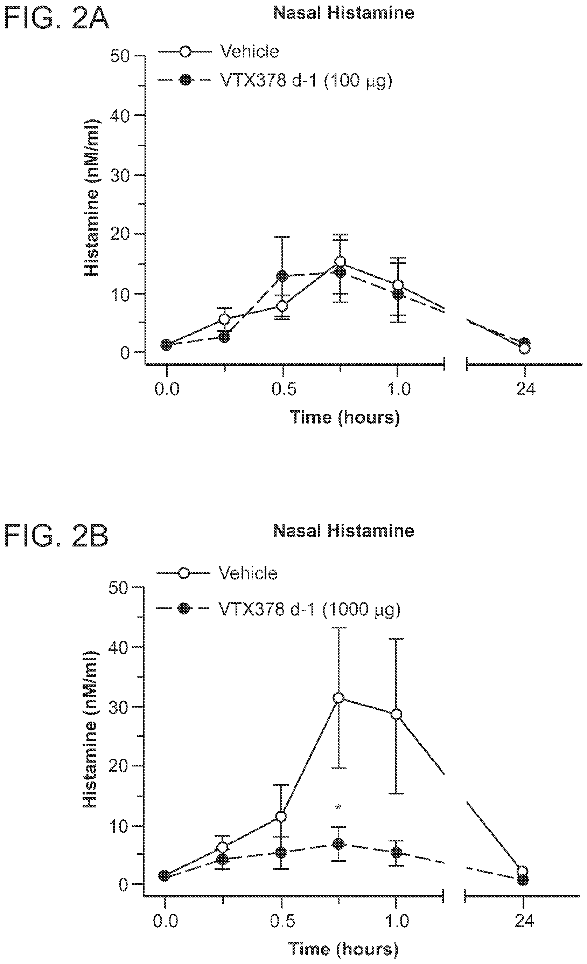

**Figure 2** is two graphs depicting that one day pretreatment with VTX-378 reduces allergic mediator, nasal histamine. Pretreatment with a single dose of VTX-378 at 1000 ug (B) administered intranasally one day prior to ragweed challenge significantly reduced the level of nasal histamine. The effect of a lower dose at 100 ug (A) was less significant.

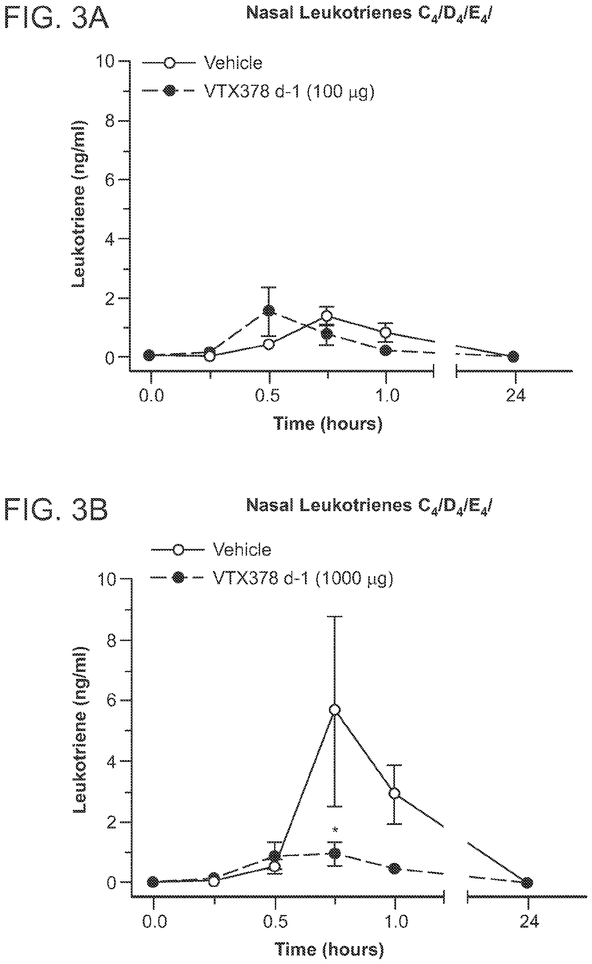

**Figure 3** is two graphs depicting that one day pretreatment with VTX-378 reduces allergic mediator, nasal leukotrienes C₄/D₄/E₄. Pretreatment with a single dose of VTX-378 at 1000 ug (B) administered intranasally one day prior to ragweed challenge significantly reduced the level of nasal leukotrienes C₄/D₄/E₄. The effect of a lower dose at 100 ug (A) was less significant.

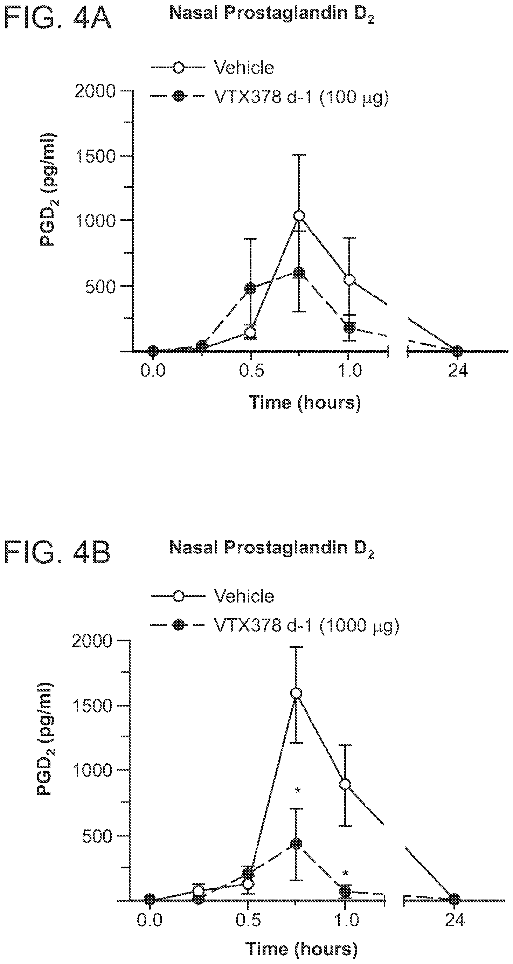

**Figure 4** is two graphs depicting that one day pretreatment with VTX-378 reduces allergic mediator, nasal prostaglandin D₂. Pretreatment with a single dose of VTX-378 at 1000 ug (B) administered intranasally one day prior to ragweed challenge significantly reduced the level of nasal prostaglandin D₂. The effect of a lower dose at 100 ug (A) was less significant.

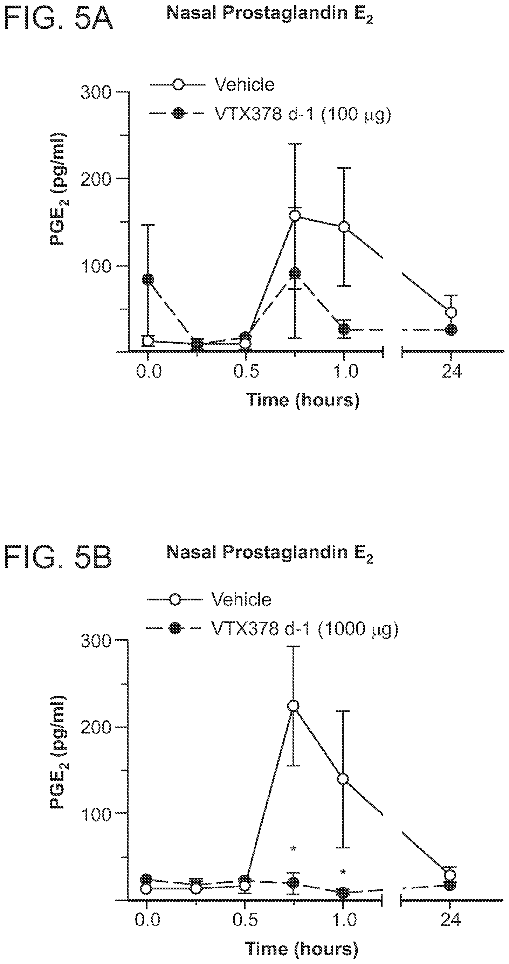

**Figure 5** is two graphs depicting that one day pretreatment with VTX-378 reduces allergic mediator, nasal prostaglandin E₂. Pretreatment with a single dose of VTX-378 at 1000 ug (B) administered intranasally one day prior to ragweed challenge significantly reduced the level of nasal prostaglandin E₂. The effect of a lower dose at 100 ug (A) was less significant.

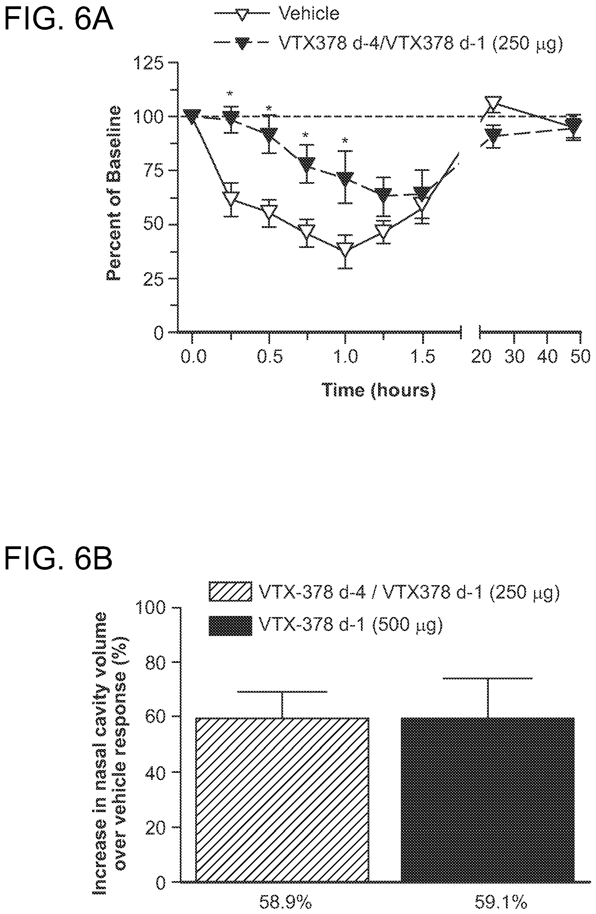

**Figure 6** is two graphs establishing that two-day pretreatment with VTX-378 reduces nasal congestion. (A) Pretreatment with two 250 ug doses of VTX-378 administered intranasally one day and four-day prior to ragweed challenge significantly attenuated allergic responses in a ragweek-sensitized beagle dog model of allergic rhinitis. (B) The effect of two-day pretreatment at 250 ug dose was similar to the effect of one-day pretreatment at 500 ug dose.

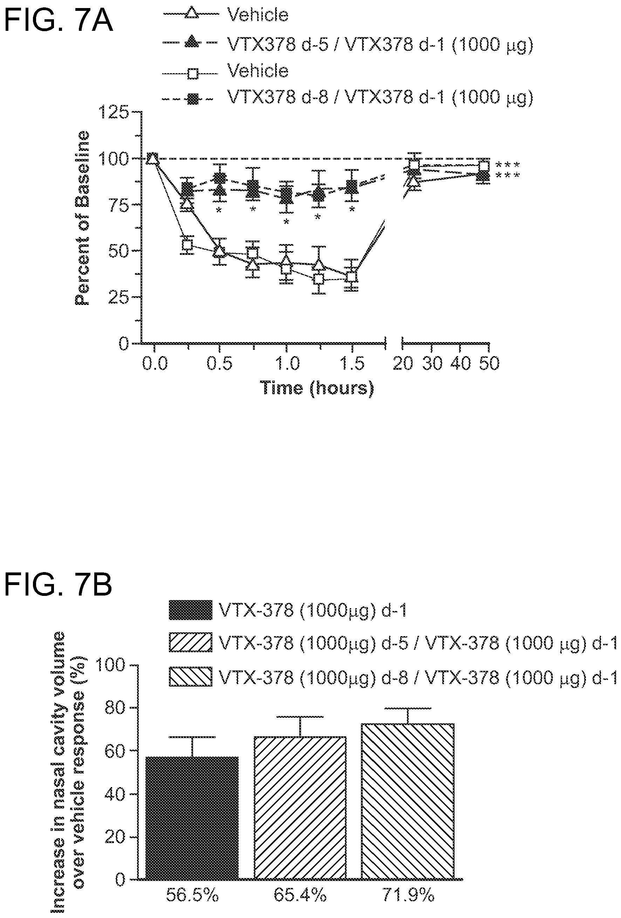

**Figure 7** is two graphs establishing that two-day pretreatment with VTX-378 reduces nasal congestion. (A) Pretreatment with two 1000 ug doses of VTX-378 administered intranasally i) one day and four-day or ii) one day and eight-day prior to ragweed challenge significantly attenuated allergic responses in a ragweek-sensitized beagle dog model of allergic rhinitis. (B) The effect of two-day pretreatment with longer interval (*i.e.,* one day and eight-day prior to ragweed challenge) was better than the effect of the one-day pretreatment or the two-day pretreatment with shorter interval (*i.e.,* one day and five-day prior to ragweed challenge).

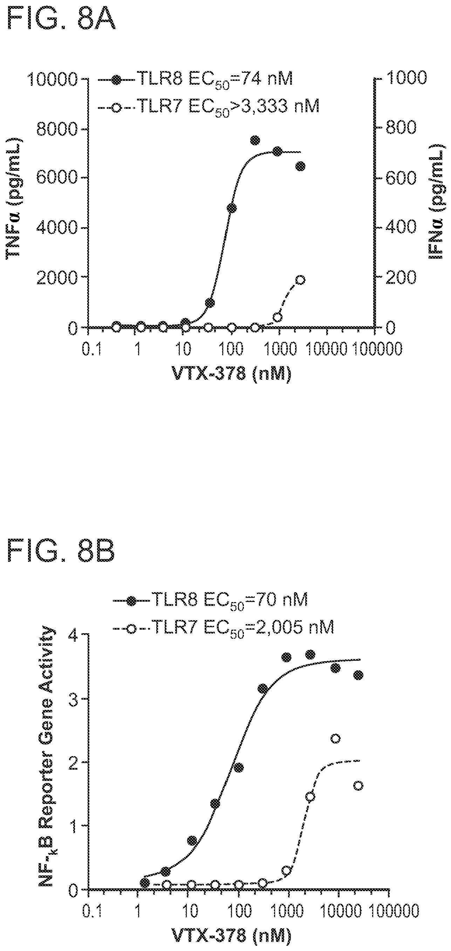

**Figure 8** is two graphs depicting the potency and selectivity of VTX-378 for TLR8. The half-maximal effective concentration (EC₅₀) for VTX-378 activation of TLR8 and TLR7 was assessed in peripheral blood mononuclear cells (PBMCs) by the amount of secreted TNFα (A) and by the activity of NF-κB (B) from 15 healthy donors and also in HEK293 cells transfected with various human TLRs. VTX-378 was a potent, selective agonist of TLR8 and had no detectable activity on TLR2, TLR3, TLR5, TLR6, or TLR9 at concentration up to 25 uM and minimal to no activity on TLR4.

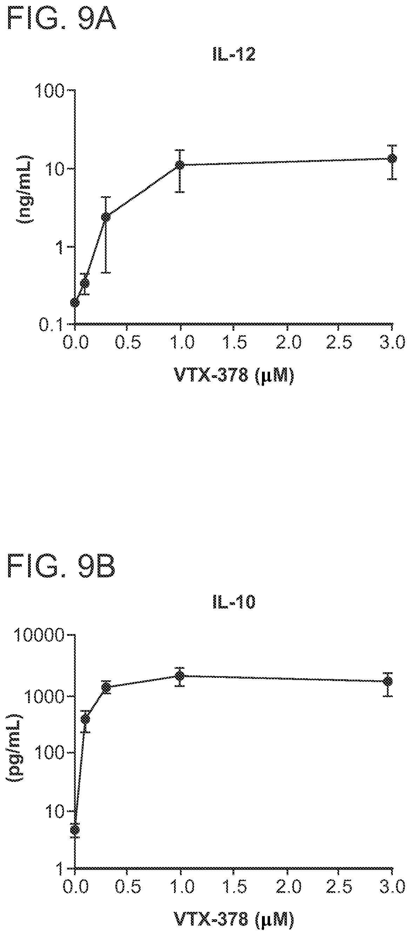

**Figure 9** is two graphs depicting that VTX-378 stimulates production of IL-12 and IL-10. Co-culture of human whole blood with VTX-378 resulted in dose dependent increase in production of IL-12 (A) and IL-10 (B).

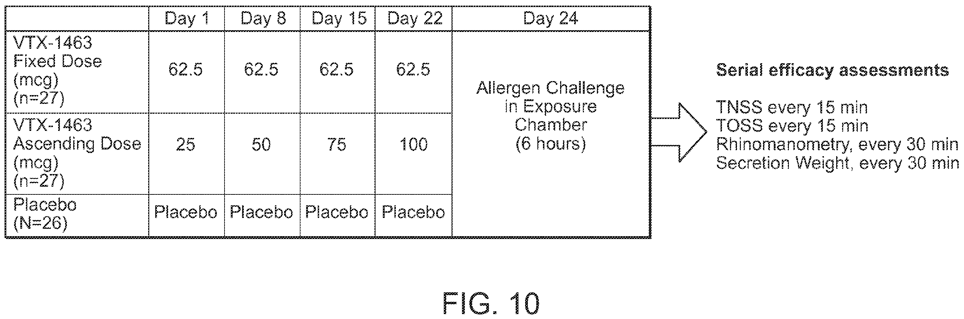

**Figure 10** is a chart depicting the allergen challenge chamber study procedures. VRXP-B103 was a randomized placebo controlled study comparing a total dose of 250 ug VTX-1463 administered weekly via the intranasal route over 4 weeks as either an ascending dose (25, 50, 75, 100 ug) or a fixed does (62.5 ug/week) to placebo. Approximately 48 hours after the last dose, subjects were exposed to grass pollen for 6 hours and serial efficacy assessments were performed.

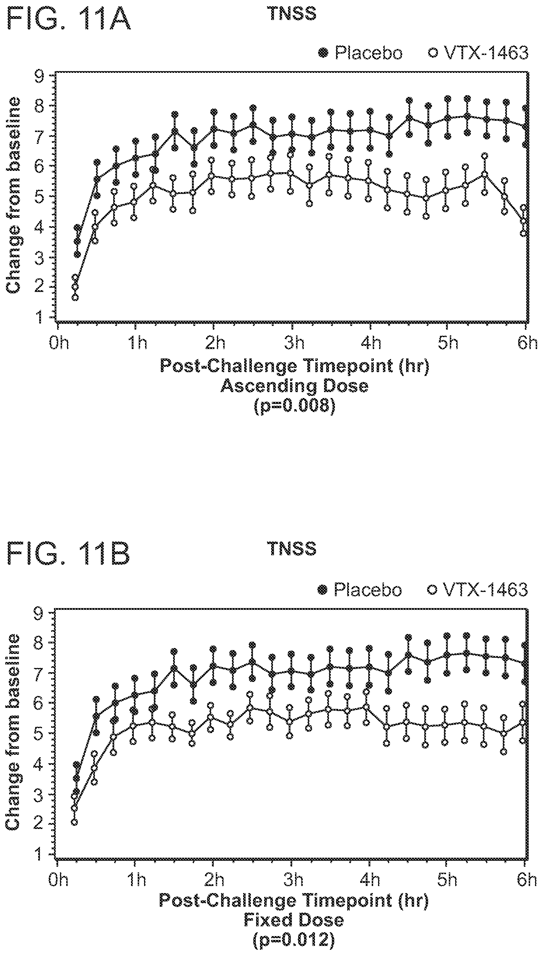

**Figure 11** is two graphs depicting the total nasal symptom scores (TNSS) following administration of VTX-1463 (an intranasal formulation of VTX-378). VTX-1463 was administered weekly via the intranasal route over 4 weeks as either an ascending dose (A) or a fixed dose (B) to placebo. Approximately 48 hours after the last dose, subjects were exposed to grass pollen for 6 hours and serial efficacy assessments were performed. TNSS is a validated endpoint which is a composite score of 4 symptoms graded by the subject on a scale from 0 to 3, nasal congestion, nasal itching, sneezing and rhinorrhea (total score range from 0-12). The higher score the worse allergic symptoms.

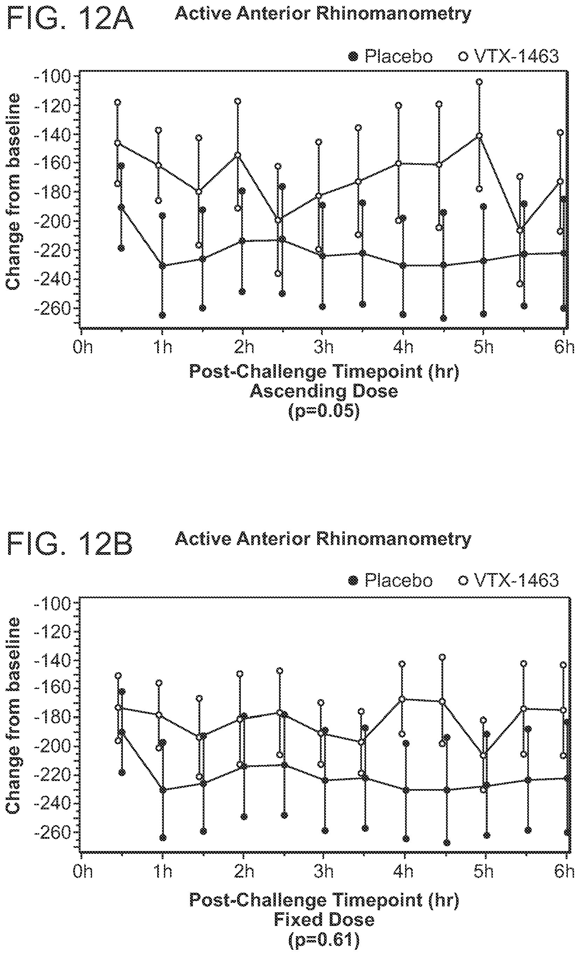

**Figure 12** is two graphs depicting nasal congestion as assessed by active anterior rhinomanometry (AAR) following administration of VTX-1463. VTX-1463 was administered weekly via the intranasal route over 4 weeks as either an ascending dose (A) or a fixed dose (B) to placebo. Approximately 48 hours after the last dose, subjects were exposed to grass pollen for 6 hours and serial efficacy assessments were performed. AAR is a direct objective measurement of nasal expiratory airflow. The lower number the worse allergic symptoms.

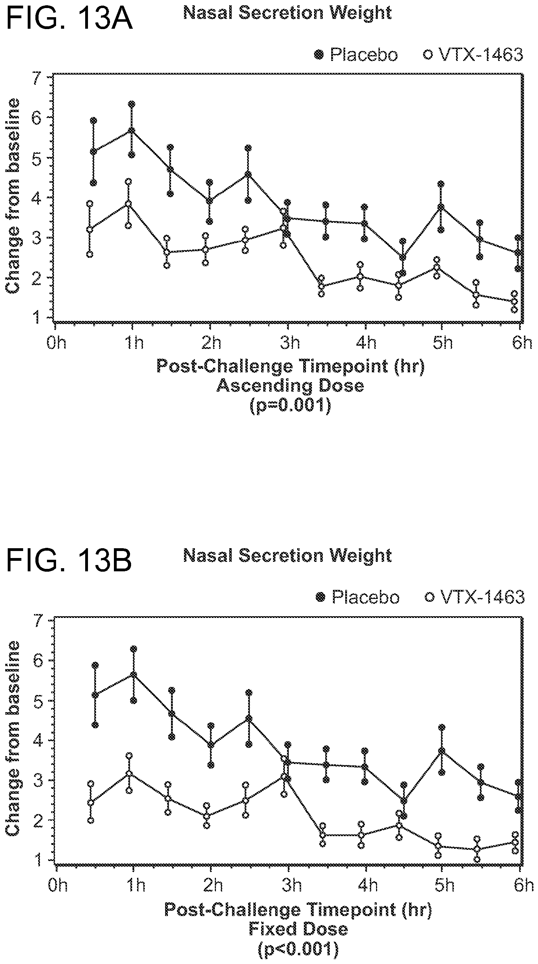

**Figure 13** is two graphs depicting the nasal secretion weight following administration of VTX-1463. VTX-1463 was administered weekly via the intranasal route over 4 weeks as either an ascending dose (A) or a fixed dose (B) to placebo. Approximately 48 hours after the last dose, subjects were exposed to grass pollen for 6 hours and serial efficacy assessments were performed. Nasal secretions were collected using sealed preweighted packets of absorbent paper tissues. The heavier the weight the worse allergic symptoms.

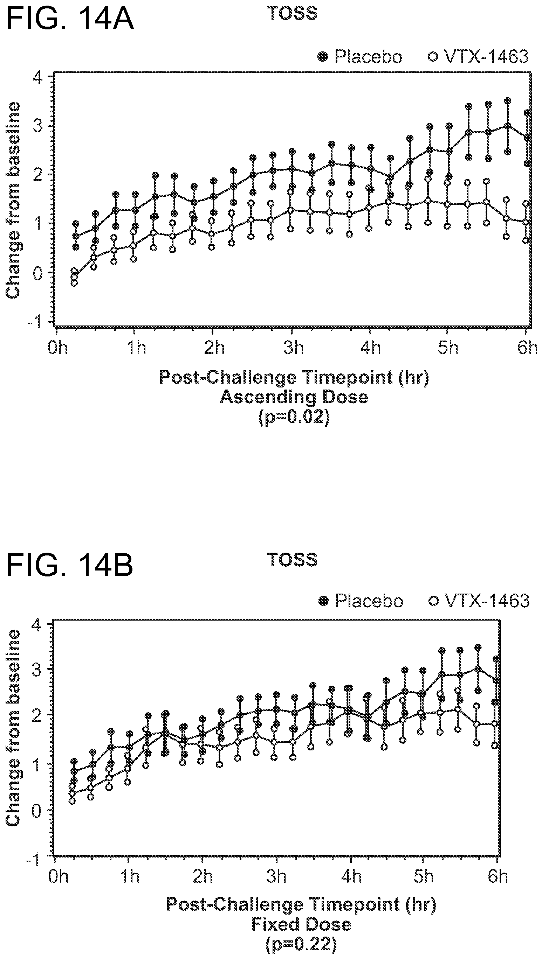

**Figure 14** is two graphs depicting the total ocular symptom scores (TOSS) following administration of VTX-1463. VTX-1463 was administered weekly via the intranasal route over 4 weeks as either an ascending dose (A) or a fixed dose (B) to placebo. Approximately 48 hours after the last dose, subjects were exposed to grass pollen for 6 hours and serial efficacy assessments were performed. TOSS is a composite score of 3 symptoms graded by the subject on a scale from 0 to 3, itchy eyes, tearing eyes, and eye redness (total score ranges from 0 to 9). The higher score the worse allergic symptoms.

### DETAILED DESCRIPTION OF THE INVENTION

The present invention provides a pharmaceutical composition comprising VTX-378 or a salt thereof for use in treating allergic disease, such as allergic rhinitis, said composition being in a form suitable for nasal administration or other routes of administration. VTX-378 is a novel, potent and selective small molecule TLR8 agonist. Formulations of VTX-378 or a salt thereof are described in PCT International Publication No. WO10/014913. VTX-1463 is an intranasal formulation of VTX-378. The formulations of the present invention are suitable for use in methods for the treatment of allergic diseases, such as allergic rhinitis, as described herein.

The present disclosure provides stable formulations of VTX-378. The formulations of are for use in the treatment of conditions pertaining to allergic responses (e.g., allergic rhinitis) in a subject as described herein, in accordance with the invention.

The present disclosure provides stable formulations of VTX-378, such as intranasal formulation. Such formulations are suitable for the pharmaceutical uses described herein below. Preferably, the formulations are solid powder formulations suitable for administration via the respiratory tract of a subject, preferably the nasal passages of human subjects.

The present invention also provides a pharmaceutical composition for use in treating, ameliorating, or preventing allergic diseases, such as allergic rhinitis, the pharmaceutical composition comprising VTX-378 or a salt or hydrate thereof to a subject in need thereof, wherein the VTX-378 is administered intranasally. The allergic diseases can be in or out of the relevant allergy season. Since VTX-378 can control both rapid onset of symptoms and longer term immunomodulation, it is for use in treating, ameliorating, or preventing seasonal or perennial allergic diseases.

A "subject" in the context of the present invention is preferably a mammal. The mammal can be a human, non-human primate, mouse, rat, dog, cat, horse, or cow, but are not limited to these examples. A subject can be male or female. A subject can be one who has been previously diagnosed or identified as having allergic diseases, and optionally has already undergone, or is undergoing, a therapeutic intervention for the allergic diseases such as allergic rhinitis. Alternatively, a subject can also be one who has not been previously diagnosed as having allergic diseases, but who is at risk of developing such condition. For example, a subject can be one who exhibits one or more symptoms for allergic diseases.

An "allergen" in the context of the present invention comprises any substance that can cause an allergy, such as, but is not limited to, bee stings, penicillin, various food allergies, pollens, animal detritus (e.g., house dust mite, cat, dog and cockroach), mold, and fungal allergens. The allergen of the present invention includes ragweed and other weeds such as curly dock, lambs quarters, pigweed, plantain, sheep sorrel and sagebrush. The allergen of the present invention also includes, but is not limited to, grass pollens, e.g., from Bermuda grass, Johnson grass, Kentucky bluegrass, Orchard grass, Sweet vernal grass, St. Augustine grass or Timothy grass. The allergen of the present invention can include, but is not limited to, tree pollens from cedar, catalpa, elm, hickory, olive, pecan, sycamorem, walnut, ash, box elder, cottonwood, date palm, maple, Phoenix palm, poplar, willow, pine tree, oak tree or birch tree.

The term "allergic disease" or "allergic condition" in the context of the present invention refers to a hypersensitivity disorder of the immune system, such as, but is not limited to, allergic rhinitis, allergic conjunctivitis, allergic bronchial asthma, atopic eczema, anaphylaxis, insect sting, drug allergies, food allergies, ocular allergic disease or multiple allergies (such as asthma, eczema and allergic rhinitis together). The allergic disease in the present invention is seasonal or perennial.

The terms "disease," "disorder" and "condition" are used interchangeably herein, and refer to any disruption of normal body function, or the appearance of any type of pathology. The etiological agent causing the disruption of normal physiology may or may not be known. Furthermore, although two patients may be diagnosed with the same disorder, the particular symptoms displayed by those individuals may or may not be identical.

The term "alleviating" or "ameliorating" as used herein refers to alleviation or reduction in at least one symptom of the disease, disorder or condition. The term encompasses the administration and/or application of one or more compounds described herein, to a subject, for the purpose of providing management of, or remedy for a condition. "Treatment" for the purposes of this disclosure, may, but does not have to, provide a cure; rather, "treatment" may be in the form of management of the condition.

The term "preventing" as used herein includes either preventing or slowing the onset of a clinically evident disease progression altogether or preventing or slowing the onset of a preclinically evident stage of a disease in individuals at risk. This includes prophylactic treatment of those at risk of developing a disease.

### TLR Agonists of the Invention

### Form ulation

The chemical structure of VTX-378 is shown below:

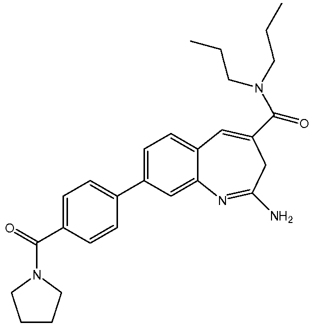

The formulations are suitable for intranasal administration to a subject, preferably a human subject.

Compositions of the present invention comprise VTX-378 or a salt thereof and one or more pharmaceutically acceptable excipients. The term excipient as used herein broadly refers to a biologically inactive substance used in combination with the active agents of the formulation. An excipient can be used, for example, as a solubilizing agent, a stabilizing agent, a diluent, an inert carrier, a preservative, a binder, a disintegrant, a coating agent, a flavoring agent, or a coloring agent. Preferably, at least one excipient is chosen to provide one or more beneficial physical properties to the formulation, such as increased stability and/or solubility of the active agent(s). VTX-378 or a salt thereof as described herein is the primary active agent in the formulations of the present invention.

A "pharmaceutically acceptable" excipient is one that has been approved by a state or federal regulatory agency for use in animals, and preferably for use in humans, or is listed in the U.S. Pharmacopia, the European Pharmacopia or another generally recognized pharmacopia for use in animals, and preferably for use in humans.

Examples of excipients include certain inert proteins such as albumins; hydrophilic polymers such as polyvinylpyrrolidone; amino acids such as aspartic acid (which may alternatively be referred to as aspartate), glutamic acid (which may alternatively be referred to as glutamate), lysine, arginine, glycine, and histidine; fatty acids and phospholipids such as alkyl sulfonates and caprylate; surfactants such as sodium dodecyl sulphate and polysorbate; nonionic surfactants such as such as TWEEN®, PLURONICS®, or polyethylene glycol (PEG); carbohydrates such as glucose, sucrose, mannose, maltose, trehalose, and dextrins, including cyclodextrins; polyols such as mannitol and sorbitol; chelating agents such as EDTA; and salt-forming counter-ions such as sodium.

The formulations of VTX-378 or a salt thereof may contain a cyclodextrin which increases the aqueous solubility of the TLR agonist. Cyclodextrins are crystalline, nonhygroscopic cyclic oligomers of α-D-glucopyranose. As a result of a lack of rotation about the bonds connecting the glucopyranose units, the cyclodextrins are not cylindrical, but toroidal in shape. Because of this restricted rotation they have a rigid structure with a central cavity whose size varies according to the number of glucopyranose units in the molecule. The three most common cyclodextrins are α-cyclodextrin, β-cyclodextrin and γ-cyclodextrin, which consist of six, seven, or eight glucopyranose units, respectively. Due to the arrangement of hydroxyl groups within the cyclodextrin molecule and the shape of the molecule, the internal surface of the cavity is hydrophobic, while the outside surface is hydrophilic. The primary hydroxyl groups are located on the narrower (inner) side of the toroidal molecule, while the secondary hydroxyl groups are located on the wider (outer) edge. This arrangement permits the cyclodextrins to accommodate a wide variety of small hydrophobic molecules within the hydrophobic cavity by forming an inclusion complex.

Suitable cyclodextrins for use in the formulations of the invention are known in the art. For example, TRAPPSOL™ and other cyclodextrins are made by CTD, Inc. (High Springs, FL), and CAPTISOL® (sulfobutylether β-cyclodextrin) is present in commercially available injectables such as ABILIFY IM™, GEODON, and VFEND IV. Preferably, CAPTISOL® is used in the formulations of the present invention.

Although cyclodextrins are the preferred solubilizing agents, other water-solubilizing agents may be used. Examples of other such agents include Poloxamer, Povidone K17, Povidone K12, Tween 80, ethanol, Cremophor/ethanol, polyethylene glycol 300, polyethylene glycol 400, and propylene glycol. In preferred embodiments, the formulations of the invention contain less than 10% v/v of such agents. In certain embodiments, oil-based solubilizing agents such as lipiodol and peanut oil, are used.

The formulations of VTX-378 may be prepared as a liquid or in a solid form such as a powder, tablet, pill or capsule. Liquid formulations may take such forms as suspensions, solutions or emulsions in oily or aqueous vehicles, and may contain formulatory agents such as suspending, stabilizing and/or dispersing agents. In one embodiment, the formulation is an aqueous solution. In another embodiment, the final formulation is lyophilized. In other embodiments, the formulation comprises a colloidal drug delivery system. Such drug delivery systems include, for example, liposomes, albumin microspheres, microemulsions, nano-particles and nanocapsules. VTX-1463 is an intranasal formulation of VTX-378.

The present invention is directed generally to formulations of pharmaceutical compositions containing a benzo[b]azepine TLR agonist. The compositions are suitable for use in the treatment of allergic reactions and other conditions that are mediated by toll-like receptors. Specifically, the present invention includes a stable powder formulation of a benzo[b]azepine TLR agonist. In preferred embodiments, the benzo[b]azepine TLR agonist is formulated into a powder suitable for administration intranasally via the respiratory tract.

Preferably, the formulations of the invention comprise benzo[b]azepine TLR agonist at a concentration of from 0.25 wt% to 10 wt%, or from 0.5 wt % to 8 wt%, or from 0.8 wt% to 5 wt%, or from 1 wt% to 4%, or from 1.5 wt% to 3 wt%. In certain embodiments, the benzo[b]azepine TLR agonist is formulated at a concentration of from about 0.5 wt% to about 5 wt%, or about 0.5 wt% to about 3 wt%, or from about 0.5 wt% to about 2 wt%. In certain embodiments, the benzo[b]azepine TLR agonist is formulated at a concentration of about 0.25 wt%, about 0.5 wt%, about 1 wt%, about 1.5 wt%, about 2 wt%, about 3 wt%, about 4 wt%, about 5 wt%, about 6 wt%, about 8 wt%, or about 10 wt%.

In some embodiments, the formulation comprises about 1-15%, 1-10%, or 1-5% weight/weight (w/w) of an acid, preferably citric acid. In certain embodiments, the formulation comprises 1%, 1.5%, 2%, 2.5%, 3%, 3.5%, 4%, 4.5%, 5%, 10%, or 15% w/w of an acid, preferably citric acid. In certain embodiments, the solid (i.e., powder) formulation comprises an acid in an amount that results when the solid is prepared from a solution that comprises the acid at a concentration of 0.5 M, or 0.8 M, or 1 M, or 1.2 M, or 1.5 M, or 2 M. Preferred such embodiments comprise citric acid. In preferred embodiments, the formulation is suitable for administration intranasally via the respiratory tract.

In some embodiments, the formulation comprises about 0.1%, 0.2%, 0.3%, 0.4%, 0.5%, 0.75%, 1%, 1.5%, 2%, 2.5%, 3%, 3.5%, 4%, 4.5%, or 5% (w/w) of a surfactant, preferably of a phospholipid. An example of a preferred phospholipids is Lipoid S-75.

The present invention further provides a pharmaceutical composition for use in the treatment of allergic reactions, wherein the composition comprises the benzo[b]azepine TLR agonist formulation of the present invention, VTX-378. In a preferred embodiment, the benzo[b]azepine TLR agonist formulation is administered in combination with one or more additional active agents.

In certain embodiments, the benzo[b]azepine TLR agonist is administered to the subject at a dose of about 0.02 to 10 mg/kg or about 0.04 to 5 mg/kg body weight of the subject. In certain embodiments, the benzo[b]azepine TLR agonist is administered at a dose of about .02 mg/kg, about .05 mg/kg, about 1 mg/kg, about 2 mg/kg, or about 5 mg/kg. In certain further embodiments, the benzo[b]azepine TLR agonist formulation is administered to the subject on a weekly or biweekly basis. In other preferred embodiments, the formulation is administered to the subject on an as-needed basis.

The present disclosure also provides a pharmaceutical pack or kit comprising one or more containers filled with a solid benzo[b]azepine TLR agonist formulation of the invention for the treatment of allergic reaction or one or more symptoms thereof. In some preferred embodiments, the formulation comprises about 1-15%, 1-10%, or 1-5% w/w of an acid, preferably citric acid. In certain embodiments, the formulation comprises 1%, 1.5%, 2%, 2.5%, 3%, 3.5%, 4%, 4.5%, 5%, 10%, or 15% w/w of an acid. In a particular embodiment, the formulation is a powder formulation of the benzo[b]azepine TLR agonist (1E, 4E)-2-amino-N,N-dipropyl-8-(4-(pyrrolidine-1-carbonyl)phenyl)-3H-benzo[b]azepine-4-carboxamide containing an acid, preferably citric acid. Preferably, the benzo[b]azepine TLR agonist is formulated at a concentration of at least 0.5 wt% and the formulation is suitable for delivery via inhalation in a mammal, preferably a human.

The present disclosure also provides for a use of a formulation of a benzo[b]azepine TLR agonist, preferably a TLR7 or a TLR8 agonist in the manufacture of a medicament for treating an allergic reaction in a subject. An example such allergic reaction is allergic rhinitis. In one embodiment, the TLR agonist is formulated at a concentration of at least 0.5 wt%. Moreover, the formulation is suitable for administration to the subject, where the subject is preferably a human, by nasal inhalation. Accordingly, administration is preferably accomplished by mucosal adsorption in the nasal passages. In certain embodiments, the TLR agonist is administered to the subject at a dose of about 0.02 to 10 mg/kg, at a dose of about 0.04 to 5 mg/kg. In certain further embodiments, the benzo[b]azepine TLR agonist formulation is administered to the subject on a weekly or biweekly basis, and in some embodiments, administration is on an as-needed basis

In a preferred embodiment, the benzo[b]azepine TLR agonist formulation is administered in combination with one or more additional active agents. Such additional active agents include antihistamines, anti-allergens, and the like.

In this specification and in the claims that follow, reference will be made to a number of terms, which shall be defined to have the definitions set forth below.

When referring to a compound of the invention, applicants intend the term "compound" to encompass not only the specified molecular entity but also its pharmaceutically acceptable, pharmacologically active analogs, including, but not limited to, salts and hydrates thereof.

The terms "treating" and "treatment" as used herein refer to reduction in severity and/or frequency of symptoms, elimination of symptoms and/or underlying cause, prevention of the occurrence of symptoms and/or their underlying cause, and improvement or remediation of damage. For example, treatment of a patient by administration of an anti-cancer agent of the invention encompasses chemoprevention in a patient susceptible to developing cancer (e.g., at a higher risk, as a result of genetic predisposition, environmental factors, or the like) and/or in cancer survivors at risk of cancer recurrence, as well as treatment of a cancer patient dual by inhibiting or causing regression of a disorder or disease.

By the terms "effective amount" and "therapeutically effective amount" of a compound of the invention is meant a nontoxic but sufficient amount of the drug or agent to provide the desired effect.

By "pharmaceutically acceptable" is meant a material that is not biologically or otherwise undesirable, i.e., the material may be incorporated into a pharmaceutical composition administered to a patient without causing any undesirable biological effects or interacting in a deleterious manner with any of the other components of the composition in which it is contained. When the term "pharmaceutically acceptable" is used to refer to a pharmaceutical carrier or excipient, it is implied that the carrier or excipient has met the required standards of toxicological and manufacturing testing or that it is included on the Inactive Ingredient Guide prepared by the U.S. Food and Drug administration. "Pharmacologically active" (or simply "active") as in a "pharmacologically active" derivative or analog, refers to a derivative or analog having the same type of pharmacological activity as the parent compound and approximately equivalent in degree.

By "as-needed," as in "as-needed administration" is meant that a formulation is administered to a patient when symptoms are observed, or when symptoms are expected to appear, or at any time that the patient and/or treating physician deems it appropriate to treat (therapeutically or prophylactically) undesirable symptoms (e.g., symptoms arising from an allergic reaction).

In preferred embodiments, the benzo[b]azepine TLR agonist formulation is a powder comprising solid particles that is suitable for administration via inhalation. The solid particles comprise the active agent (i.e., benzo[b]azepine TLR agonist), a carrier, optionally an acid, optionally a surfactant, and optionally additional excipients. The powder may be prepared by the methods described in the Examples below, or by any convenient method. An example of a preparatory method is spray drying a solution containing the active agent (and other components) onto a powder comprising the carrier compound. Another example is freeze drying a solution comprising all of the components of the final powder.

For example, the formulation is a powder suitable for administration to the nasal passage of a patient. In some embodiments, the formulation further comprises carrier particles such as lactose. In some embodiments, the formulation further comprises an acid. In some embodiments, the formulation further comprises a surfactant.

For example, the formulation is sterile.

For example, agonist is formulated at a concentration of from about 0.25 wt% to about 10 wt%. For example, the agonist is formulated at a concentration of from about 1 wt% to about 5 wt%. For example, the agonist is formulated at a concentration of from about 2 wt% to about 4 wt%. For example, the agonist is formulated at a concentration of at least 2 wt%.

In one embodiment, the agonist is to be administered by the respiratory tract. For example, the agonist is to be administered by nasal inhalation.

In one embodiment, the agonist is administered to the subject as needed.

For example, the agonist is to be administered in combination with one or more other active agents, such as anti-allergen, an antiviral agent, an antifungal agent, or an antibacterial agent.

For example, a formulation including the agonist of the invention is suitable for administration to the subject by inhalation.

For example, the agonist of the invention is administered in combination with one or more other treatment modalities.

For example the allergic reaction to be treated by the method of the invention is caused by an allergen selected from pollen, dust, or a pollutant.

It will be appreciated that the precise therapeutic dose of the VTX-378 or a salt thereof will depend on the age and condition of the patient and the nature of the condition to be treated and will be at the ultimate discretion of the attendant physician.

The VTX-378 or a salt thereof may conveniently be presented in unit dose form. A convenient unit dose formulation for intranasal administration contains the active ingredient in an amount of from 5 microgram (mcg) to 1000 mcg, preferably in the range of 10 to 500 mcg, most preferably 25 to 100 mcg, which may be administered to either one or both nostrils.

A preferred unit dose formulation may be provided as a single dose in a sealed unit, for example a vial of glass or plastics material which may be filled and sealed using conventional manufacturing techniques. Alternatively, a sealed vial of plastics material may be produced by form-fill-seal technology. Preferably the vial and the components of the pharmaceutical formulation filled therein are heat stable. The sealed vial may be sterilized, for example by autoclaving at 121 °C for not less than 15 minutes, to provide a sterile unit dosage vial which can be assembled into a convenient delivery device prior to use.

The formulations are optionally contained in unit-dose or multi-dose containers, for example, in sealed ampules or vials, and may be in a lyophilized condition. Examples of unit dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for intranasal administration to a patient, including suspensions (e.g., aqueous or non aqueous liquid suspensions, oil in water emulsions, or a water in oil liquid emulsions), solutions, and elixirs; liquid dosage forms suitable for subcutaneous administration to a subject; and sterile solids (e.g., crystalline or amorphous solids) that can be reconstituted to provide liquid dosage forms suitable for intranasal administration to a subject.

In an alternative aspect of the present invention there is provided a pharmaceutical composition in a form adapted for intranasal administration which comprises VTX-378 or a salt thereof, which solution has a pH in the range of pH 5 to pH 7.

In certain embodiments, compositions of the present invention further comprise one or more adjuvants. Examples of suitable adjuvants include potentiators of the immune response such as microbial derivatives (e.g., bacterial products, toxins such as cholera toxin and heat labile toxin from E. coli, lipids, lipoproteins, nucleic acids, peptidogylcans, carbohydrates, peptides), cells, cytokines, (e.g., dendritic cells, IL-12, and GM-CSF), hormones, and small molecules. Adjuvants contemplated include, but are not limited to, oil-based adjuvants (e.g., Freund's adjuvant), CpG oligonucleotides, aluminum salt adjuvants, calcium salt adjuvants, emulsions and surfactant-based formulations (e.g., MF59, ASO2, montanide, ISA-51, ISA-720, and QA21).

Additional information with regard to the methods of making the compositions and formulations and the ingredients comprising the compositions and formulations in accordance with the present invention can be found in standard references in the field, such as for example, "Remington's Pharmaceutical Sciences," Mack Publishing Co., Easton, PA.

### Methods of Use

Provided herein is a pharmaceutical composition for use in treating, ameliorating, or preventing allergic diseases, such as allergic rhinitis. The allergic diseases can be in or out of allergy season. The allergic diseases can also be seasonal or perennial. One discovery of the present invention is that VTX-378 is a novel, selective, and potent TLR8 agonist with an EC₅₀ around 100 nM (Figure 8). Incubation with VTX-378 (the active pharmaceutical ingredient in VTX-1463; VTX-1463 is an intranasal formulation of VTX-378) stimulates PBMCs to produce a number of Th-1 cytokines and chemokines including IL-10, IL-12, IFN, MCP-1 and MIP-1β (Figure 9). Another discovery of the present invention is that VTX-378 significantly reduces the allergic response to antigen challenge in a subject with superior efficacy. VTX-378 also provides both rapid onset of symptom control and longer term immunomodulation. For example, VTX-378 significantly reduces production of allergic mediators in ragweed-sensitized beagle dogs, a well characterized model of allergic rhinitis (Figures 1-7). VTX-378 also attenuates allergic rhinitis symptoms in atopic subjects exposed to grass allergen. VTX-1463 administered weekly over 4 weeks as either an ascending dose (25, 50, 75, 100 mcg) or a fixed dose (62.5 mcg/week) to placebo significantly improves total nasal symptom score (TNSS), significantly improves airflow response to allergen challenge as measured by active anterior rhinomanometry (AAR), reduces allergen induced secretion weights and significantly improves the total ocular symptom score (TOSS) compared to placebo (Figures 11-14). In addition to its superior efficacy, VTX-1463 is also safe and well tolerated in subjects.

The locally administered TLR8 agonist provides immediate/early benefit via modulation of mast cell responses. The weekly dosing regimen is a great convenience compared to existing daily or twice daily treatment schemes for antihistamines and intranasal steroids.

VTX-378 provides a novel option for allergy subjects with rapid onset of symptom control and longer term immunomodulation, thereby treating, ameliorating, or preventing both seasonal and perennial allergic diseases.

In some embodiments, the allergic disease refers to, but is not limited to, allergic rhinitis, allergic conjunctivitis, allergic bronchial asthma, atopic eczema, anaphylaxis, insect sting, drug allergies, food allergies, multiple allergies (such as asthma, eczema and allergic rhinitis together), or any combination thereof. For example, the allergic disease is allergic rhinitis.

In some embodiments, the allergic disease is caused by an allergen. The allergen includes, but is not limited to, pollen, weed, bee venom, insect venom, penicillin, food allergy, animal detritus, mold, a fungal allergen, or any combination thereof. In a preferred embodiment, the allergen is tree pollen. For example, the tree pollen is from, but is not limited to, cedar, catalpa, elm, hickory, olive, pecan, Sycamore, walnut, ash, box elder, cottonwood, date palm, maple, Phoenix palm, poplar, willow, pine tree, oak tree, birch tree, or any combination thereof. In another preferred embodiment, the allergen is grass pollen. For example, the grass pollen is, but is not limited to, Bermuda grass pollen, Johnson grass pollen, Kentucky bluegrass pollen, Orchard grass pollen, Sweet vernal grass pollen, St. Augustine grass pollen, Timothy grass pollen, or any combination thereof. In another preferred embodiment, the allergen is a weed. The weed can be, but is not limited to, ragweed, curly dock, lambs quarter, pigweed, plantain, sheep sorrel, sagebrush, or any combination thereof.

### Combination Therapy

Combination therapy encompasses, in addition to the administration of VTX-378 or a salt thereof, the adjunctive use of one or more modalities that aid in the prevention or treatment of allergic diseases, such as allergic rhinitis. Such modalities include, but are not limited to, immunotherapeutics, anti-angiogenic agents, cytokines, hormones, antibodies, polynucleotides, photodynamic therapeutic agents, non-steroid anti-inflammatory medications, anti-histamines, alpha- adrenergic agonists, steroids and any combination thereof. As used herein, "in combination with" means that VTX-378 is administered as part of a treatment regimen that comprises one or more additional treatment modalities as described in more detail in the following sections.

In certain embodiments, VTX-378 or a salt thereof is administered prior to, concurrently with, or subsequent to the administration of the one or more other modalities. In one embodiment VTX-378 or a salt thereof is formulated with one or more other modalities. In another embodiment, the one or more other modalities are administered in a separate pharmaceutical composition. In accordance with this embodiment, the one or more other modalities may be administered to a subject by the same or different routes of administration as those used to administerVTX-378 or a salt thereof.

### Combination with therapeutic antibodies

In another embodiment, VTX-378 or a salt thereof is administered in combination with one or more immunotherapeutic agents, such as an antibody or a vaccine.

Non-limiting examples of therapeutic and prophylactic antibodies that can be used in combination with VTX-1463include SYNAGIS® (MedImmune, MD) which is a humanized anti-respiratory syncytial virus (RSV) monoclonal antibody for the treatment of RSV infection. Other examples are humanized anti-CD18 F(ab')₂ (Genentech); CDP860 which is a humanized anti-CD18 F(ab')₂ (Celltech, UK); PRO542 which is an anti-HIV gp120 antibody fused with CD4 (Progenics/Genzyme Transgenics); Ostavir which is a human anti-Hepatitis B virus antibody (Protein Design Lab/Novartis); PROTOVIR™ which is a humanized anti-CMV IgG1 antibody (Protein Design Lab/Novartis); MAK-195 (SEGARD) which is a murine anti-TNF-α F(ab')₂ (Knoll Pharma/BASF); IC14 which is an anti-CD14 antibody (ICOS Pharm); a humanized anti-VEGF IgG1 antibody (Genentech); OVAREX™ which is a murine anti-CA 125 antibody (Altarex); PANOREX™ which is a murine anti-17-IA cell surface antigen IgG2a antibody (Glaxo Wellcome/Centocor); BEC2 which is a murine anti-idiotype (GD3 epitope) IgG antibody (ImClone System); IMC-C225 which is a chimeric anti-EGFR IgG antibody (ImClone System); VITAXIN™ which is a humanized anti-αVβ3 integrin antibody (Applied Molecular Evolution/MedImmune); Campath 1H/LDP-03 which is a humanized anti-CD52 IgG1 antibody (Leukosite); Smart M195 which is a humanized anti-CD33 IgG antibody (Protein Design Lab/Kanebo); RITUXAN™ which is a chimeric anti-CD20 IgG1 antibody (IDEC Pharm/Genentech, Roche/Zettyaku); LYMPHOCIDE™ which is a humanized anti-CD22 IgG antibody (Immunomedics); Smart ID10 which is a humanized anti-HLA antibody (Protein Design Lab); ONCOLYM™ (Lym-1) is a radiolabelled murine anti-HLA DIAGNOSTIC REAGENT antibody (Techniclone); ABX-IL8 is a human anti-IL8 antibody (Abgenix); anti-CD11a is a humanized IgG1 antibody (Genentech/Xoma); ICM3 is a humanized anti-ICAM3 antibody (ICOS Pharm); IDEC-114 is a primatized anti-CD80 antibody (IDEC Pharm/Mitsubishi); ZEVALIN™ is a radiolabelled murine anti-CD20 antibody (IDEC/Schering AG); IDEC-131 is a humanized anti-CD40L antibody (IDEC/Eisai); IDEC-151 is a primatized anti-CD4 antibody (IDEC); IDEC-152 is a primatized anti-CD23 antibody (IDEC/Seikagaku); SMART anti-CD3 is a humanized anti-CD3 IgG (Protein Design Lab); 5G1.1 is a humanized anti-complement factor 5 (C5) antibody (Alexion Pharm); D2E7 is a humanized anti-TNF-α antibody (CAT/BASF); CDP870 is a humanized anti-TNF-α Fab fragment (Celltech); IDEC-151 is a primatized anti-CD4 IgG1 antibody (IDEC Pharm/SmithKline Beecham); MDX-CD4 is a human anti-CD4 IgG antibody (Medarex/Eisai/Genmab); CDP571 is a humanized anti-TNF-α IgG4 antibody (Celltech); LDP-02 is a humanized anti-α4β7 antibody (LeukoSite/Genentech); OrthoClone OKT4A is a humanized anti-CD4 IgG antibody (Ortho Biotech); ANTOVA™ is a humanized anti-CD40L IgG antibody (Biogen); ANTEGREN™ is a humanized anti-VLA-4 IgG antibody (Elan); MDX-33 is a human anti-CD64 (FcγR) antibody (Medarex/Centeon); SCH55700 is a humanized anti-IL-5 IgG4 antibody (Celltech/Schering); SB-240563 and SB-240683 are humanized anti-IL-5 and IL-4 antibodies, respectively, (SmithKline Beecham); rhuMab-E25 is a humanized anti-IgE IgG1 antibody (Genentech/Norvartis/Tanox Biosystems); ABX-CBL is a murine anti CD-147 IgM antibody (Abgenix); BTI-322 is a rat anti-CD2 IgG antibody (Medimmune/Bio Transplant); Orthoclone/OKT3 is a murine anti-CD3 IgG2a antibody (ortho Biotech); SIMULECT™ is a chimeric anti-CD25 IgG1 antibody (Novartis Pharm); LDP-01 is a humanized anti-β₂-integrin IgG antibody (LeukoSite); Anti-LFA-1 is a murine anti CD18 F(ab')₂ (Pasteur-Merieux/Immunotech); CAT-152 is a human anti-TGF-β₂ antibody (Cambridge Ab Tech); and Corsevin M is a chimeric anti-Factor VII antibody (Centocor). The above-listed immunoreactive reagents, as well as any other immunoreactive reagents, may be administered according to any regimen known to those of skill in the art, including the regimens recommended by the suppliers of the immunoreactive reagents.

### Combination with immunoregulatory agents

In certain embodiments, VTX-378 or a salt thereof is administered in combination with an immunoregulatory agent. In some embodiments, VTX-378 or a salt thereof is formulated with the immunoregulatory agent. An "immunoregulatory agent" is a substance that suppresses, masks, or enhances the immune system of the subject to whom it is administered. Exemplary agents are those that suppress cytokine production, downregulate or suppress self-antigen expression, or mask the MHC antigens. Examples of such agents include 2-amino-6-aryl-5-substituted pyrimidines (*see,* U.S. Pat. No. 4,665,077), azathioprine (or cyclophosphamide, if there is an adverse reaction to azathioprine); bromocryptine; glutaraldehyde (which masks the MHC antigens, as described in U.S. Pat. No. 4,120,649); anti-idiotypic antibodies for MHC antigens and MHC fragments; cyclosporin A; steroids such as glucocorticosteroids, *e*.*g*., prednisone, methylprednisolone, and dexamethasone; cytokine or cytokine receptor antagonists including anti-interferon-γ, -β, or -α antibodies; anti-tumor necrosis factor-α antibodies; anti-tumor necrosis factor-β antibodies; anti-interleukin-2 antibodies and anti-IL-2 receptor antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain; streptokinase; TGF-β; streptodornase; FK506; RS-61443; deoxyspergualin; and rapamycin. Examples of cytokines include, but are not limited to lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α; mullerian-inhibiting substance; mouse gonadotropin-associated peptide; inhibin; activin; vascular endothelial growth factor; integrin; thrombopoiotin (TPO); nerve growth factors such as NGF-α; platelet-growth factor; transforming growth factors (TGFs) such as TGF-α and TGF-α; insulin-like growth factor-I and -II; erythropoietin (EPO); osteoinductive factors; interferons; colony stimulating factors (CSFs) such as macrophage-CSF (M-CSF); granulocyte-macrophage-CgP (GM-CSP); and granulocyte-CSF (G-CSF); interleukins (Ils) such as IL-1, IL-la, IL-2, 1L-3, IL-4, IL-5, IL-6, IL-7, IL-8, IL-9, IL-1 I, IL-12, IL-15; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

In certain embodiments, the methods further include administering to the subject one or more immunomodulatory agents, preferably a cytokine. Preferred cytokines are selected from the group consisting of interleukin-1 (IL-1), IL-2, IL-3, IL-12, IL-15, IL-18, G-CSF, GM-CSF, thrombopoietin, and γ interferon.

### Combination with compounds that enhance monocyte or macrophage function

In certain embodiments, a compound that enhances monocyte or macrophage function (*e.g.,* at least about 25%, 50%, 75%, 85%, 90%, 9% or more) can be used in conjunction with VTX-378 or a salt thereof. Such compounds are known in the art and include, without limitation, cytokines such as interleukins (*e.g.,* IL-12), and interferons (*e.g.,* alpha or gamma interferon).

In certain embodiments, the compound that enhances monocyte or macrophage function is formulated with VTX-378 and is thus administered concurrently with VTX-378 or a salt thereof.

In other embodiments, the compound that enhances monocyte or macrophage function is administered separately from VTX-378 or a salt thereof and can be administered concurrently (within a period of hours of each other), during the same course of therapy, or sequentially with VTX-378 or a salt thereof. In such embodiments, the compound that enhances monocyte or macrophage function is preferably administered to a human subject. In one embodiment, the human subject has a blood leukocyte, monocyte, neutrophil, lymphocyte, and/or basophil count that is within the normal range for humans. Normal ranges for human blood leukocytes (total) is about 3.5-10.5 (10⁹/L). Normal ranges for human blood neutrophils is about 1.7-7.0 (10⁹/L), monocytes is about 0.3-0.9 (10⁹/L), lymphocytes is about 0.9-2.9 (10⁹/L), basophils is about 0-0.3 (10⁹/L), and eosinophils is about 0.05-0.5 (10⁹/L). In other embodiments, the human subject has a blood leukocyte count that is less than the normal range for humans, for example at least about 0.01, 0.05, 0.1, 0.2, 0.3, 0.4, 0.5, 0.6, 0.7, or 0.8 (10⁹/L) leukocytes.

### Administration and Dosing

VTX-378 of the invention is administered intranasally. This disclosure also refers to administration by an oral, a parenteral, an intraperitoneal, an intravenous, an intraarterial, a transdermal, a sublingual, an intramuscular, a rectal, a transbucal, a liposomal, an inhalation, a vaginal, an intraocular, via local delivery by catheter or stent, a subcutaneous, an intraadiposal, an intraarticular, an intrathecal, or an intradermal route, or in a slow release dosage form.

The formulations of the present invention contain an amount of VTX-378 that is effective for the intended use. It will be appreciated that the precise therapeutic dose of the VTX-378 or a salt thereof will depend on the age and condition of the patient and the nature of the condition to be treated and will be at the ultimate discretion of the attendant physician. Particular dosages are also selected based on a number of other factors including the age, sex, species and condition of the patient. Effective amounts can also be extrapolated from dose-response curves derived from *in vitro* test systems or from animal models.

A preferred unit dose formulation may be provided as a single dose in a sealed unit, for example a vial of glass or plastics material which may be filled and sealed using conventional manufacturing techniques. Alternatively, a sealed vial of plastics material may be produced by form-fill-seal technology. Preferably the vial and the components of the pharmaceutical formulation filled therein are heat stable. The sealed vial may be sterilized, for example by autoclaving at 121 °C for not less than 15 minutes, to provide a sterile unit dosage vial which can be assembled into a convenient delivery device prior to use.

In an alternative aspect of the present invention there is provided a pharmaceutical composition in a form adapted for intranasal administration which comprises VTX-378 or a salt thereof, which solution has a pH in the range of pH 5 to pH 7.

The dose of VTX-378 is measured in units of mcg or mg per dose administered to a patient. Any measurement of dose can be used in conjunction with the compositions and methods of the invention and dosage units can be converted by means standard in the art. Exemplary doses of VTX-378 include microgram amounts per dose of the subject. Preferably, each dose is from about 25 mcg to 1000 mcg. Alternatively, a single dose is 1500 mcg, 2000 mcg, 2500 mcg, or 3000 mcg.

Examples of dosing regimens that can be used in the methods of the invention include, but are not limited to, daily, three times weekly (intermittent), weekly, or every 14 days. In certain embodiments, dosing regimens include, but are not limited to, monthly dosing or dosing every 6-8 weeks. VTX-378 of the present invention is administered by intranasal administration weekly or biweekly alone or in combination with a suitable treatment modality for the treatment or prevention of allergic diseases in a subject, preferably a human subject.

### Kits

The present disclosure also provides a pharmaceutical pack or kit comprising one or more containers filled with VTX-378 or a salt thereof in a form suitable for nasal administration or other routes of administration.

In certain embodiments, the kit further comprises instructions for use in the treatment or prevention of allergic disease, as well as side effects and dosage information for one or more routes of administration. Optionally associated with such container(s) is a notice in the form prescribed by a governmental agency regulating the manufacture, use or sale of pharmaceuticals or biological products, which notice reflects approval by the agency of manufacture, use or sale for human administration.

The invention is further defined by reference to the following examples, which are not meant to limit the scope of the present invention.

### EXAMPLES

### Example 1. In Vitro Characterization of Selectivity, Potency, and Pharmacodynamics of VTX-1463.

Preclinical studies were performed with VTX-378, the active pharmaceutical ingredient in VTX-1463. The specificity of VTX-378 for human TLR8 relative to other TLRs was assessed in transfected Human Embryonic Kidney 293 (HEK293) cells engineered to express individual TLRs (2-9) via transfection, and assessed using an NF-κB driven reporter system. As shown in Figure 8, VTX-378 was a potent activator of TLR8 with a half maximal effective concentration (EC₅₀) of ∼100 nM and a weak activator of TLR7 with an EC₅₀ of ∼2000 nM in this test system. VTX-378 had no detectable activity on TLR2, TLR3, TLR5, TLR6 or TLR9 and minimal to no activity on TLR4. The pharmacologic effects of VTX-378 were characterized *in vitro* using isolated human peripheral blood mononuclear cells (PBMCs). Incubation with VTX-378 stimulated PBMCs to produce a number of Th-1 cytokines and chemokines including IL-12, IFN, MCP-1 and MIP-1β. VTX-378 also stimulated production of IL-10 as shown in Figure 9 and Table 1. The overall pattern of cytokine induction, notably including the production of IL-12, is distinct for TLR8 stimulation compared to other human TLRs (Table 2).

**Table 1. VTX-378 stimulates production of IL-12 and IL-10.**

| | Mean Mediator Level (pg/mL) ± SEM | | | | EC50 (uM) | | |
|---|---|---|---|---|---|---|---|
| Analyte | Control | VTX-378 (1.6uM) | Imiquimod (25 uM) | CpG (1.5 uM) | VTX-378 | Imiquimod | CpG |
| IL-10 | 0.9±0.6 | 72±29 | 59±24 | 5.5±2.9 | 0.12 | 17.0 | 0.01 |
| IL-12p40 | 5.7±2.2 | 140±32* | 10.2±3.5 | 5.5±3.1 | 0.23 | 0.27 | N/C |
| IL-12p70 | <0.5 | 38±10 | 0.6±0.6 | 0.6±0.6 | 0.22 | N/C | N/C |
| *p<0.05 to Control, N/C= not calculated | | | | | | | |

**Table 2. Comparison of TLR7, TLR8 and TLR9.**

| | TLR7 | TLR8 | TLR9 |
|---|---|---|---|
| Expression | Plasmacytoid DC | Myeloid DC Monocytes | Plasmacytoid DC B cells |
| Natural ligand | ssRNA | ssRNA | Bacterial DNA |
| IFNα | Yes | No | Yes |
| TNFα | No | Yes | No |
| IL-12 | No | Yes | No |

### Example 2. Efficacy of VTX-1463 in a Dog Model of Allergic Rhinitis.

The therapeutic activity of VTX-378 was assessed in a well characterized model of allergic rhinitis in ragweed-sensitized beagle dogs (Figures 1-7). Acoustic rhinometry (AcR) can be used to measure the direct effect on the nasal mucosa following nasal instillation of ragweed antigen. In multiple experiments using ragweed-sensitized dogs, intranasal administration of 100-1000 mcg of VTX-378 produced a reproducible, dose-dependent, statistically significant reduction in the allergic response to antigen challenge (Figure 1). VTX-378 significantly reduced the production of allergic mediators, such as nasal histamine, nasal leukotrienes C₄/D₄/E₄, nasal prostaglandin D₂, and nasal prostaglandin E₂ (Figures 2-5). Interestingly, the responses to VTX-378 were rapid-occurring after a single dose administered 24 hours before the intranasal ragweed challenge. Studies in this model have also found that two VTX-378 doses are more effective than a single dose (Figures 6 and 7). Specifically, a single dose of 1000 mcg of VTX-378 attenuated nasal congestion 56.5% ± 10.0% while two doses separated by 4 or 7 days improved congestion 65.4% ± 10.3 and 71.9% ± 7.7%, respectively. These findings suggest that weekly intranasal administration of a TLR8 agonist may have a unique pharmacologic profile in the treatment or prevention of allergic rhinitis.

### Example 3. VTX-1463 Clinical Studies

### 1. Overview of the Clinical Trials Performed

VTX-1463 has been evaluated in three randomized, placebo controlled trials. Study VRXP-B101 was a first-in-man dose escalation study that evaluated the safety, tolerability, and pharmacokinetics of VTX-1463 in 37 healthy volunteers. Studies VRXP-B102 and VRXP-B103 were designed to assess the safety and efficacy of VTX-1463 in attenuating allergic rhinitis symptoms in atopic subjects exposed to grass allergen. Study VRXP-B102 evaluated 2 weekly doses of VTX-1463 out of allergy season and Study VRXP-B103 evaluated 4 weekly doses of VTX-1463 during allergy season. Both studies were conducted in the Vienna Challenge Chamber (VCC) (Vienna, Austria). The VCC offers a controlled and controllable paradigm in which to reproducibly evaluate the effects of medications on allergic rhinitis. This model allows the control of environmental conditions and can therefore eliminate some of the confounding factors encountered in normal outdoor studies such as the variability of the general environmental allergen load, variability with respect to the time patients spend indoors, and occurrence of concomitant respiratory tract infections. The efficacy assessments performed in the VCC include Total Nasal Symptom Score (TNSS; the sum of scores for nasal congestion, nasal itching, sneezing and rhinorrhea), nasal airflow resistance as measured by active anterior rhinomanometry (AAR), Total Ocular Symptom Score (TOSS; the sum of scores for watery eyes, itchy eyes, and red eyes), Miscellaneous Allergy Symptom Score (MASS; the sum of scores for cough, itchy throat, itchy ears and palate), and nasal secretion weight.

### 2. VRXP-B101: Phase 1 Trial in Healthy Volunteers

VRXP-B101 was a randomized, placebo controlled, two-part study to evaluate the safety, tolerability, and pharmacokinetics of intranasal administration of VTX-1463 to healthy volunteers (n=37). The first part of the study assessed cohorts exposed to increasing single doses of VTX-1463 ranging from 32 mcg to 500 mcg. The second part of the study evaluated different cohorts exposed to two doses of 250 mcg and 500 mcg separated by 7 days. Pharmacokinetic analysis demonstrated a mean Tmax occurring between 0.3 and 0.5 hours and a mean half-life (t1/2) ranging between 1.0 and 1.6 hours after intranasal dosing. For the 32 mcg and 64 mcg dose groups, quantifiable levels of VTX-1463 were below the level of detection (0.1 ng/mL). Measurable levels of VTX-1463 were present in the plasma for an average of 1.8, 2.4 and 4.0 hours following administration of the 125 mcg, 250 and 500 mcg doses, respectively.

Overall, VTX-1463 was generally safe and well tolerated. The majority of adverse events were mild (Grade 1), local (nasal), and resolved spontaneously. Local findings included runny nose, nasal irritation, nasal congestion and throat irritation.

### 3. VRXP-B102: Allergen Challenge in Atopic Individuals

VRXP-B102 was a randomized, placebo controlled multiple dose level study conducted in the VCC. The primary goal of the study was to assess the safety and efficacy of VTX-1463 in attenuating allergic rhinitis symptoms in atopic subjects exposed to grass allergen. Seventy asymptomatic or minimally symptomatic subjects with confirmed atopy to grass pollen were randomized to receive 2 weekly intranasal doses of 100, 250, or 500 mcg of VTX-1463 or placebo out of pollen season. Twenty-four hours after the second dose, subjects had baseline evaluations performed and then underwent a grass allergen challenge in the exposure chamber for 6 hours. While in the allergen exposure chamber, subjects underwent serial evaluations for symptom scores (TNSS, MASS, TOSS), active anterior rhinomanometry, spirometry and nasal secretion weight.

VTX-1463 at the doses and dose regimen studied showed improvement over placebo in attenuating allergic rhinitis symptoms. The clinical benefit of VTX-1463 appeared greatest in the 250 mcg group, where there was approximately 25% improvement based on TNSS measurements relative to placebo (p=0.071) and approximately 50% improvement in nasal airflow (AAR) compared to placebo (p=0.019). There was no apparent benefit following 2 doses of 100 mcg VTX 1463.

Adverse events were generally local, mild, and transient in nature. Local findings included runny nose, nasal irritation, nasal congestion and throat irritation. Adverse events appeared to be dose related and less severe with the second dose as compared with the first.

### 4. VRXP-B103: Allergen Challenge in Atopic Individuals in Season

Study VRXP-B103 was a randomized, placebo controlled trial in atopic individuals performed in the VCC. VRXP-B103 differed from VRXP-B102 in that the individual doses administered were much lower and that it was designed to assess the efficacy of VTX-1463 during allergy season while environmental grass pollen counts were elevated. Study VRXP-B103, compared a total dose of 250 mcg VTX-1463 administered weekly over 4 weeks as either an ascending dose (25, 50, 75, 100 mcg) or a fixed dose (62.5 mcg/week) to placebo (Figure 10). These dose regimens were selected with the goal of improving the tolerability of VTX-1463 and based on the observations in study VRXP-B102 that the 250 mcg dose level of VTX-1463 was efficacious and that the second dose was better tolerated that the first dose. Eighty asymptomatic or minimally symptomatic subjects with confirmed atopy to grass pollen were enrolled in the study and underwent a 6-hour grass allergen challenge in the VCC 48 hours after the fourth dose.

Subjects who were treated with VTX-1463 and underwent allergen challenge had significantly improved TNSS compared to placebo (p=0.012) (Figure 11). This benefit was observed with both the ascending dose (p=0.008) and fixed dose regimens (p=0.012). Mean percent change form baseline in airflow response to allergen challenge as measured by AAR was improved 20.4% in subjects that received VTX-1463 compared to subjects that received placebo (Figure 12). Clinical benefit was also observed for reduction in allergen induced secretion weights compared to placebo (p=0.001), and trended favorably for the TOSS (Figure 14) and MASS endpoints (Figure 13).

The tolerability of VTX-1463 was also improved in Study VRXP-B103. The overall incidence of AEs was approximately 5.8% higher in subjects treated with VTX-1463 than those treated with placebo. Most adverse events were local and transient and the frequency and severity of AEs did not appear to increase with cumulative number of doses or dose level.

### 5. Conclusion

VTX-1463 is a novel, selective, TLR8 agonist administered intranasally on a weekly basis for the treatment or prevention of allergic rhinitis. This product is given as a stand-alone agent and is not co-administered with allergen. Data from two, independent placebo controlled studies in subjects with grass pollen allergy performed in the VCC demonstrated clinic benefit. VTX-1463 provides near-term symptom relief, and has the potential (based on the activation of TLR8 on dendritic cells and monocytes in the nasal mucosa) to provide longer-term immunomodulation to seasonal (and/or perennial) allergens.

The locally administered TLR8 agonist provides immediate/early benefit via modulation of mast cell responses, via local IL-10 production (consistent with the preclinical data and the observation of clinical improvement in human clinical trials after 2 or 4 doses) and that additional doses (via the recurrent production of IL-12) will modulate local T cell responses and decrease Th2 tone.

The weekly dosing regimen is a great convenience compared to existing daily or twice daily treatment schemes for antihistamines and intranasal steroids.

In summary, VTX-1463 has shown superior activity in two, independent studies of allergic subjects- both in and out of allergy season. The target for this agent-TLR8-has distinct immunological properties (including IL-12 production) compared to other TLRs studied in the setting of allergy. VTX-1463 provides a new option for allergy subjects with rapid onset of symptom control and longer term immunomodulation, thereby treating, ameliorating, or preventing both seasonal and perennial allergic diseases.

## Claims

1. A pharmaceutical composition for use in a method for treating, ameliorating, or preventing an allergic disease in a subject in need thereof, the pharmaceutical composition comprising VTX-378 represented by the structure: 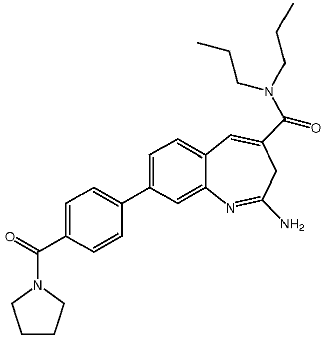 or a pharmaceutically acceptable salt or hydrate thereof, wherein VTX-378 or a pharmaceutically acceptable salt or hydrate thereof is administered intranasally.

2. The pharmaceutical composition for use of claim 1, wherein VTX-378 or a pharmaceutically acceptable salt or hydrate thereof is administered daily, three times weekly (intermittent), two times weekly, weekly, every 14 days, monthly or every 6-8 weeks.

3. The pharmaceutical composition for use of claim 1, wherein VTX-378 or a pharmaceutically acceptable salt or hydrate thereof is administered intranasally in a single dose form.

4. The pharmaceutical composition for use of claim 3, wherein said single dose of VTX-378 is from about 25 microgram (mcg) to about 1000 mcg.

5. The pharmaceutical composition for use of claim 3, wherein said single dose of VTX-378 is 1500 mcg, 2000 mcg, 2500 mcg, or 3000 mcg.

6. The pharmaceutical composition for use of claim 1, wherein the allergic disease is selected from the group consisting of allergic rhinitis, allergic conjunctivitis, allergic bronchial asthma, atopic eczema, anaphylaxis, insect sting, drug allergy, food allergy, multiple allergies and any combination thereof, preferably wherein the allergic disease is seasonal or perennial.

7. The pharmaceutical composition for use of claim 1, wherein the allergic disease is caused by an allergen, preferably wherein the allergen is selected from the group consisting of pollen, weed, bee venom, insect venom, penicillin, food allergy, animal detritus, mold, a fungal allergen, and any combination thereof.

8. The pharmaceutical composition for use of claim 7, wherein the allergen is tree pollen.

9. The pharmaceutical composition for use of claim 8, wherein the tree pollen is selected from cedar, catalpa, elm, hickory, olive, pecan, Sycamore, walnut, ash, box elder, cottonwood, date palm, maple, Phoenix palm, poplar, willow, pine tree, oak tree, birch tree, and any combination thereof.

10. The pharmaceutical composition for use of claim 7, wherein the allergen is grass pollen.

11. The pharmaceutical composition for use of claim 10, wherein the grass pollen is selected from Bermuda grass pollen, Johnson grass pollen, Kentucky bluegrass pollen, Orchard grass pollen, Sweet vernal grass pollen, St. Augustine grass pollen, Timothy grass pollen, and any combination thereof.

12. The pharmaceutical composition for use of claim 7, wherein the allergen is a weed.

13. The pharmaceutical composition for use of claim 12, wherein the weed is selected from ragweed, curly dock, lambs quarter, pigweed, plantain, sheep sorrel, sagebrush, and any combination thereof.

14. The pharmaceutical composition for use of claim 1, further comprising administering one or more modalities that aid in the prevention or treatment of said allergic disease, wherein said one or more modalities are administered prior to, concurrently with, or subsequent to the administration of VTX-378.

15. The pharmaceutical composition for use of claim 14, wherein said one or more modalities are selected from the group consisting of immunotherapeutics, anti-angiogenic agents, cytokines, hormones, antibodies, polynucleotides, photodynamic therapeutic agents, non-steroid anti-inflammatory medications, anti-histamines, alpha- adrenergic agonists, steroids, and any combination thereof.

## Patentansprüche

1. Pharmazeutische Zusammensetzung zur Verwendung in einem Verfahren zur Behandlung, Verbesserung oder Prävention einer allergischen Erkrankung bei einem Subjekt, das diese benötigt, wobei die pharmazeutische Zusammensetzung VTX-378 umfasst, dargestellt durch die Struktur: 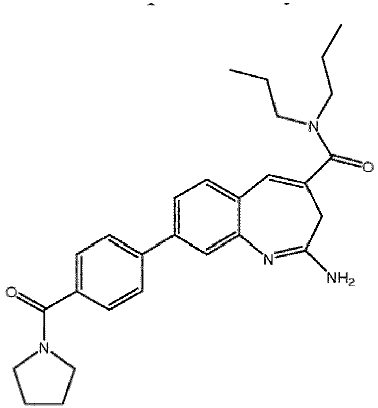 oder pharmazeutisch akzeptables Salz oder Hydrat davon, wobei VTX-378 oder ein pharmazeutisch akzeptables Salz oder Hydrat davon intranasal verabreicht wird.

2. Pharmazeutische Zusammensetzung zur Verwendung nach Anspruch 1, wobei VTX-378 oder ein pharmazeutisch akzeptables Salz oder Hydrat davon täglich, dreimal wöchentlich (intermittierend), zweimal wöchentlich, wöchentlich, alle 14 Tage, monatlich oder alle 6-8 Wochen verabreicht wird.

3. Pharmazeutische Zusammensetzung zur Verwendung nach Anspruch 1, wobei VTX-378 oder ein pharmazeutisch akzeptables Salz oder Hydrat davon intranasal in einer Einzeldosisform verabreicht wird.

4. Pharmazeutische Zusammensetzung zur Verwendung nach Anspruch 3, wobei die VTX-378-Einzeldosis zwischen etwa 25 Mikrogramm (µg) und etwa 1000 µg beträgt.

5. Pharmazeutische Zusammensetzung zur Verwendung nach Anspruch 3, wobei die VTX-378-Einzeldosis 1500 µg, 2000 µg, 2500 µg oder 3000 µg beträgt.

6. Pharmazeutische Zusammensetzung zur Verwendung nach Anspruch 1, wobei die allergische Erkrankung ausgewählt ist aus der Gruppe bestehend aus allergischer Rhinitis, allergischer Konjunktivitis, allergischem Bronchialasthma, atopischem Ekzem, Anaphylaxie, Insektenstich, Arzneimittelallergie, Nahrungsmittelallergie, mehrfachen Allergien und einer Kombination daraus, wobei die allergische Erkrankung vorzugsweise saisonal oder mehrjährig ist.

7. Pharmazeutische Zusammensetzung zur Verwendung nach Anspruch 1, wobei die allergische Erkrankung durch ein Allergen verursacht wird, wobei das Allergen vorzugsweise ausgewählt ist aus der Gruppe bestehend aus Pollen, Kraut, Bienengift, Insektengift, Penicillin, Nahrungsmittelallergie, tierischen Abfällen, Schimmel, einem Pilzallergen und einer Kombination davon.

8. Pharmazeutische Zusammensetzung zur Verwendung nach Anspruch 7, wobei das Allergen Baumpollen ist.

9. Pharmazeutische Zusammensetzung zur Verwendung nach Anspruch 8, wobei der Baumpollen ausgewählt ist aus Zeder, Trompetenbaum, Ulme, Hickory, Olive, Pekannuss, Bergahorn, Walnuss, Esche, Eschenahorn, Amerikanische Pappel, Dattelpalme, Ahorn, Phönix-Palme, Pappel, Weide, Kiefer, Eiche, Birke und einer Kombination davon.

10. Pharmazeutische Zusammensetzung zur Verwendung nach Anspruch 7, wobei das Allergen Graspollen ist.

11. Pharmazeutische Zusammensetzung zur Verwendung nach Anspruch 10, wobei der Graspollen ausgewählt ist aus Bermudagras-Pollen, Wilde Mohrenhirse-Pollen, Wiesen-Rispengras-Pollen, Knaulgras-Pollen, Ruchgras-Pollen, St. Augustingras-Pollen, Lieschgras-Pollen und einer Kombination davon.

12. Pharmazeutische Zusammensetzung zur Verwendung nach Anspruch 7, wobei das Allergen ein Kraut ist.

13. Pharmazeutische Zusammensetzung zur Verwendung nach Anspruch 12, wobei das Kraut ausgewählt ist aus Beifuß-Ambrosie, Krausem Ampfer, Weißem Gänsefuß, Geißfuß, Breitwegerich, Ackersauerklee, Salbeistrauch und einer Kombination davon.

14. Pharmazeutische Zusammensetzung zur Verwendung nach Anspruch 1, ferner umfassend die Verabreichung von einer oder mehreren Modalitäten, die zur Prävention oder Behandlung der allergischen Erkrankung beitragen, wobei die eine oder mehreren Modalitäten vor, gleichzeitig mit oder nach der Verabreichung von VTX-378 verabreicht werden.

15. Pharmazeutische Zusammensetzung zur Verwendung nach Anspruch 14, wobei die eine oder mehreren Modalitäten ausgewählt sind aus der Gruppe bestehend aus Immuntherapien, anti-angiogenen Mitteln, Zytokinen, Hormonen, Antikörpern, Polynucleotiden, photodynamischen therapeutischen Wirkstoffen, nichtsteroidalen entzündungshemmenden Arzneimitteln, Antihistaminen, alphaadrenergen Agonisten, Steroiden und einer Kombination davon.

## Revendications

1. Composition pharmaceutique destinée à être utilisée dans une méthode pour le traitement, l'amélioration ou la prévention d'une maladie allergique chez un sujet qui en a besoin, la composition pharmaceutique comprenant du VTX-378 représenté par la structure : 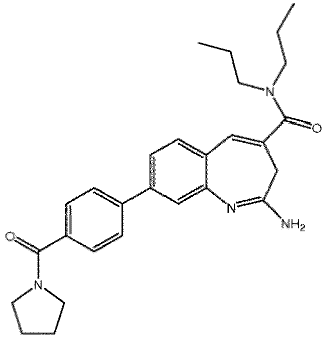 ou un sel pharmaceutiquement acceptable ou hydrate de celui-ci, du VTX-378 ou un sel pharmaceutiquement acceptable ou hydrate de celui-ci étant administré par voie intranasale.

2. Composition pharmaceutique destinée à être utilisée selon la revendication 1, du VTX-378 ou un sel pharmaceutiquement acceptable ou hydrate de celui-ci étant administré une fois par jour, trois fois par semaine (de façon intermittente), deux fois par semaine, une fois par semaine, tous les 14 jours, une fois par mois ou toutes les 6-8 semaines.

3. Composition pharmaceutique destinée à être utilisée selon la revendication 1, du VTX-378 ou un sel pharmaceutiquement acceptable ou hydrate de celui-ci étant administré par voie intranasale sous une forme de dose unitaire.

4. Composition pharmaceutique destinée à être utilisée selon la revendication 3, ladite dose unitaire de VTX-378 étant d'environ 25 microgrammes (µg) à environ 1000 µg.

5. Composition pharmaceutique destinée à être utilisée selon la revendication 3, ladite dose unitaire de VTX-378 étant de 1500 µg, 2000 µg, 2500 µg ou 3000 µg.

6. Composition pharmaceutique destinée à être utilisée selon la revendication 1, la maladie allergique étant choisie dans le groupe constitué par la rhinite allergique, la conjonctivite allergique, l'asthme bronchique allergique, l'eczéma atopique, l'anaphylaxie, une piqûre d'insecte, une allergie médicamenteuse, une allergie alimentaire, de multiples allergies et une quelconque association de ceux-ci, de préférence la maladie allergique étant saisonnière ou apériodique.

7. Composition pharmaceutique destinée à être utilisée selon la revendication 1, la maladie allergique étant provoquée par un allergène, de préférence l'allergène étant choisi dans le groupe constitué par du pollen, une mauvaise herbe, du venin d'abeille, du venin d'insecte, la pénicilline, une allergie alimentaire, des détritus d'animaux, une moisissure, un allergène fongique et une quelconque association de ceux-ci.

8. Composition pharmaceutique destinée à être utilisée selon la revendication 7, l'allergène étant du pollen d'arbres.

9. Composition pharmaceutique destinée à être utilisée selon la revendication 8, le pollen d'arbres étant choisi entre le cèdre, le catalpa, l'orme, l'hickory, l'olivier, le pacanier, le sycomore, le noyer, le frêne, l'érable négondo, *Populus* sect. *Aigeiros,* le palmier dattier, l'érable, le palmier Phoenix, le peuplier, le saule, le pin, le chêne, le bouleau et une quelconque association de ceux-ci.

10. Composition pharmaceutique destinée à être utilisée selon la revendication 7, l'allergène étant du pollen de plantes herbacées.

11. Composition pharmaceutique destinée à être utilisée selon la revendication 10, le pollen de plantes herbacées étant choisi entre le pollen de chiendent pied-de-poule, le pollen de sorgho d'Alep, le pollen de pâturin des prés, le pollen de dactyle aggloméré, le pollen de flouve odorante, le pollen de faux kikuyu, le pollen de fléole des prés et une quelconque association de ceux-ci.

12. Composition pharmaceutique destinée à être utilisée selon la revendication 7, l'allergène étant une mauvaise herbe.

13. Composition pharmaceutique destinée à être utilisée selon la revendication 12, la mauvaise herbe étant choisie entre l'ambroisie, le rumex crépu, le chénopode blanc, l'amarante, le plantain, la petite oseille, l'armoise et une quelconque association de ceux-ci.

14. Composition pharmaceutique destinée à être utilisée selon la revendication 1, comprenant en outre l'administration d'une ou plusieurs modalités qui aident à la prévention ou au traitement de ladite maladie allergique, ladite ou lesdites modalités étant administrées avant l'administration de VTX-378, en même temps que celle-ci ou après celle-ci.

15. Composition pharmaceutique destinée à être utilisée selon la revendication 14, ladite ou lesdites modalités étant choisies dans le groupe constitué par les agents immunothérapeutiques, les agents anti-angiogéniques, les cytokines, les hormones, les anticorps, les polynucléotides, les agents thérapeutiques photodynamiques, les médicaments anti-inflammatoires non stéroïdiens, les antihistaminiques, les agonistes alpha-adrénergiques, les stéroïdes et une quelconque association de ceux-ci.
